# FASCICULE DE BREVET EUROPEEN

(11) **EP 1 353 912 B1**
(45) Date de publication et mention de la délivrance du brevet: **24.08.2005**
(21) Numéro de dépôt: 02700318.5
(22) Date de dépôt: 11.01.2002
(51) Int. Cl.: C07D 277/42, C07D 417/06, C07D 417/14, A61K 31/425

(54) **DERIVES DE 2-ARYLIMINO-2,3-DIHYDROTHIAZOLES, LEURS PROCEDES DE PREPARATION ET LEUR UTILISATION THERAPEUTIQUE**
2-ARYLIMINO-2,3-DIHYDROTHIAZOL DERIVATE, VERFAHREN ZU IHRER HERSTELLUNG UND IHRE THERAPEUTISCHE VERWENDUNG
2-ARYLIMINO-2,3-DIHYDROTHIAZOLE DERIVATIVES, METHODS FOR PREPARING THEM AND THERAPEUTIC USE THEREOF

(30) Priorité: 12.01.2001 FR 0100396
(43) Date de publication de la demande: 22.10.2003
(73) Titulaire: SOCIETE DE CONSEILS DE RECHERCHES ET D'APPLICATIONS SCIENTIFIQUES (S.C.R.A.S.), 75016 Paris (FR)
(72) Inventeur: MOINET, Christophe, Montreal, Quebec H7V 4A7 (FR); SACKUR, Carole, F-75004 Paris (FR); THURIEAU, Christophe, F-75116 Paris (FR)
(74) Mandataire: Bourgouin, André
(86) Numéro de dépôt international: PCT/FR2002/000093
(87) Numéro de publication internationale: WO 2002/055510

(56) Documents cités:
- EP-A- 0 023 964
- WO-A-01/07424
- WO-A-97/00868
- FR-A- 1 347 371
- OMAR A M M E ET AL: "Synthesis and biological evaluation of new 2,3-dihydrothiazole derivatives for antimicrobial, antihypertensive, and anticonvulsant activities" JOURNAL OF PHARMACEUTICAL SCIENCES,US,AMERICAN PHARMACEUTICAL ASSOCIATION. WASHINGTON, vol. 73, no. 8, août 1984 (1984-08), pages 1166-1168, XP002136746 ISSN: 0022-3549

## Description

La présente demande a pour objet de nouveaux dérivés de 2-arylimino-2,3-dihydrothiazoles et leurs procédés de préparation. Ces produits ont une bonne affinité avec certains sous-types de récepteurs de la somatostatine et présentent donc d'intéressantes propriétés pharmacologiques. L'invention concerne également ces mêmes produits en tant que médicaments, des compositions pharmaceutiques les contenant et leur utilisation pour la préparation d'un médicament destiné à traiter les états pathologiques ou les maladies dans lesquels un (ou plusieurs) des récepteurs de la somatostatine est (sont) impliqué(s).

La somatostatine (SST) est un tétradécapeptide cyclique qui a été isolé pour la première fois de l'hypothalamus en tant que substance inhibitrice de l'hormone de croissance (Brazeau P. et al., *Science* 1973, **179,** 77-79). Elle intervient également en tant que neurotransmetteur dans le cerveau (Reisine T. et al., *Neuroscience* 1995, 67, 777-790 ; Reisine et al., *Endocrinology* 1995, **16,** 427-442). Le clonage moléculaire a permis de montrer que la bioactivité de la somatostatine dépend directement d'une famille de cinq récepteurs liés à la membrane.

L'hétérogénéité des fonctions biologiques de la somatostatine a conduit à des études pour essayer d'identifier les relations structure-activité des analogues peptidiques sur les récepteurs de la somatostatine, ce qui a amené la découverte de 5 sous-types de récepteurs (Yamada et al., *Proc. Natl. Acad. Sci. U.S.A,* **89,** 251-255, 1992 ; Raynor, K. et al, *Mol. Pharmacol.,* **44,** 385-392, 1993). Les rôles fonctionnels de ces récepteurs sont actuellement activement étudiés. Les affinités avec les différents sous-types de récepteurs de la somatostatine ont été associés au traitement des désordres / maladies suivants. L'activation des sous-types 2 et 5 a été associée à la suppression de l'hormone de croissance (GH) et plus particulièrement à celle des adénomes sécrétant GH (acromégalie) et de ceux sécrétant l'hormone TSH. L'activation du sous-type 2 mais pas du sous-type 5 a été associée au traitement des adénomes sécrétant la prolactine. D'autres indications associées avec l'activation des sous-types de récepteurs de la somatostatine sont la resténose, l'inhibition de la sécrétion d'insuline et/ou de glucagon et en particulier le diabète mellitus, l'hyperlipidémie, l'insensiblité à l'insuline, le Syndrome X, l'angiopathie, la rétinopathie proliférative, le phénomène de Dawn et la néphropathie; l'inhibition de la sécrétion d'acide gastrique et en particulier les ulcères peptiques, les fistules entérocutanées et pancréaticocutanées, le syndrome du colon irritable, le syndrome de Dumping, le syndrome des diarrhées aqueuses, les diarrhées reliées au SIDA, les diarrhées induites par la chimiothérapie, la pancréatite aiguë ou chronique et les tumeurs gastrointestinales sécrétrices; le traitement du cancer comme les hépatomes; l'inhibition de l'angiogénèse, le traitement des désordres inflammatoires comme l'arthrite; le rejet chronique des allogreffes; l'angioplastie; la prévention des saignements des vaisseaux greffés et des saignements gastrointestinaux. Les agonistes de la somatostatine peuvent aussi être utilisés pour diminuer le poids d'un patient.

Parmi les désordres pathologiques associés à la somatostatine (Moreau J.P. et al., *Life Sciences,* 1987, **40,** 419 ; Harris A.G. et al., *The European Journal of Medicine*, **1993,** 2, 97-105), on peut donc citer par exemple : l'acromégalie, les adénomes hypophysaires, la maladie de Cushing, les gonadotrophinomes et les prolactinomes, les effets secondaires cataboliques des glucocorticoïdes, le diabète insulinodépendant, la rétinopathie diabétique, la néphropathie diabétique, l'hyperthyroïdie, le gigantisme, les tumeurs gastroentéropancréatiques endocriniennes dont le syndrome carcinoïde, le VIPome, l'insulinome, la nésidioblastose, l'hyperinsulinémie, le glucagonome, le gastrinome et le syndrome de Zollinger-Ellison, le GRFome ainsi que le saignement aigu des varices oesophagiennes, le reflux gastrooesophagien, le reflux gastroduodénal, la pancréatite, les fistules entérocutanées et pancréatiques mais aussi les diarrhées, les diarrhées réfractaires du syndrome d'immunodépression acquise, la diarrhée chronique sécrétoire, la diarrhée associée avec le syndrome de l'intestin irrité, les troubles liés au peptide libérateur de gastrine, les pathologies secondaires aux greffes intestinales, l'hypertension portale ainsi que les hémorragies des varices chez des malades avec cirrhose, l'hémorragie gastro-intestinale, l'hémorragie de l'ulcère gastroduodénale, la maladie de Crohn, les scléroses systémiques, le dumping syndrome, le syndrome du petit intestin, l'hypotension, la sclérodermie et le carcinome thyroïdien médullaire, les maladies liées à l'hyperprolifération cellulaire comme les cancers et plus particulièrement le cancer du sein, le cancer de la prostate, le cancer thyroïdien ainsi que le cancer pancréatique et le cancer colorectal, les fibroses et plus particulièrement la fibrose du rein, la fibrose du foie, la fibrose du poumon, la fibrose de la peau, également la fibrose du système nerveux central ainsi que celle du nez et la fibrose induite par la chimiothérapie, et d'autres domaines thérapeutiques comme, par exemple, les céphalées y compris les céphalées associées aux tumeurs hypophysaires, les douleurs, les accès de panique, la chimiothérapie, la cicatrisation des plaies, l'insuffisance rénale résultant d'un retard de croissance, l'obésité et retard de croissance lié à l'obésité, le retard de croissance utérin, la dysplasie du squelette, le syndrome de Noonan, le syndrome d'apnée du sommeil, la maladie de Graves, la maladie polykystique des ovaires, les pseudokystes pancréatiques et ascites, la leucémie, le méningiome, la cachexie cancéreuse, l'inhibition des H pylori, le psoriasis ainsi que la maladie d'Alzheimer. On peut également citer l'ostéoporose.

La déposante a trouvé que les composés de formules générales (i) et (xv) décrits ci-après présentaient une affinité et une sélectivité pour les récepteurs de la somatostatine. Comme la somatostatine et ses analogues peptidiques ont souvent une mauvaise biodisponibilité par voie orale et une faible sélectivité (Robinson, C., *Drugs of the Future,* 1994, **19,** 992; Reubi, J.C. et al., *TIPS,* 1995, **16,** 110), lesdits composés, agonistes ou antagonistes non-peptidiques de la somatostatine, peuvent être avantageusement utilisés pour traiter les états pathologiques ou les maladies tels que présentés ci-dessus et dans lesquels un (ou plusieurs) des récepteurs de la somatostatine est (sont) impliqué(s). De manière préférentielle, lesdits composés peuvent être utilisés pour le traitement de l'acromégalie, des adénomes hypophysaires ou des tumeurs gastroentéropancréatiques endocriniennes dont le syndrome carcinoïde.

La demande de brevet PCT WO 01/07424, antérieure à la date de priorité de la présente demande mais publiée postérieurement à ladite date de priorité, décrit des composés répondant à la formule générale (I) 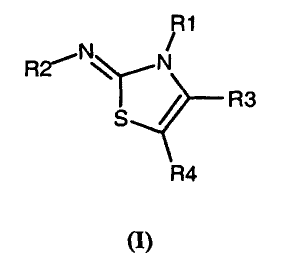 sous forme racémique, d'énantiomère ou toutes combinaisons de ces formes, dans laquelle :
R1 représente un radical amino(C₂-C₇)alkyle, aminoalkylarylalkyle, aminoalkylcycloalkylalkyle, (C₁-C₁₅)alkyle, (C₃-C₇)cycloalkyle, (C₁-C₆)alkyl(C₃-C₆)cycloalkyle, (C₃-C₆)cycloalkylalkyle, cyclohexénylalkyle, alkényle, alkynyle, aryle carbocyclique comptant au moins deux cycles dont l'un au moins n'est pas aromatique, aralkyle carbocyclique ou hétérocyclique éventuellement substitué sur le groupe aryle, *bis*-arylalkyle, alkoxyalkyle, furannylalkyle, tétrahydrofurannylalkyle, dialkylaminoalkyle, N-acétamidoalkyle, cyanoalkyle, alkylthioalkyle, arylhydroxyalkyle, aralkoxyalkyle, morpholinoalkyle, pyrrolidinoalkyle, pipéridinoalkyle, N-alkylpyrrolidinoalkyle, N-alkylpipérazinylalkyle ou oxopyrrolidinoalkyle,
ou R1 représente l'un des radicaux représentés ci-dessous : 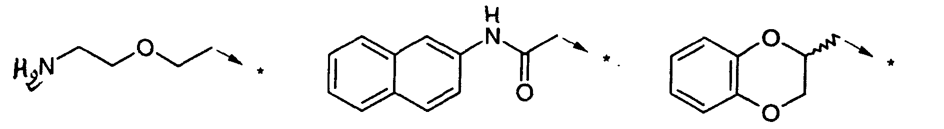 ou encore R1 représente un radical -C(R11)(R12)-CO-R10 ;
R2 représente un radical aryle carbocyclique ou hétérocyclique éventuellement substitué,
   ou bien R2 représente l'un des radicaux représentés ci-dessous : 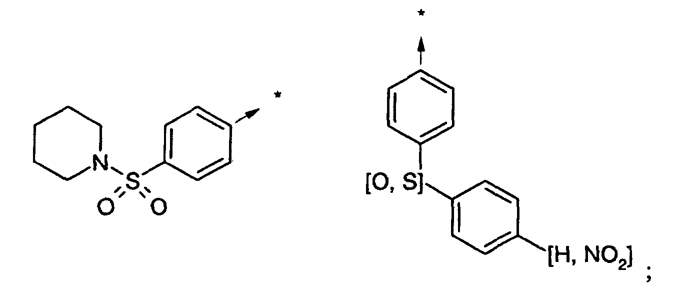
R3 représente un radical alkyle, adamantyle, aryle carbocyclique ou hétérocyclique éventuellement substitué, aralkyle carbocyclique ou hétérocyclique éventuellement substitué sur le groupe aryle,
ou R3 représente l'un des radicaux représentés ci-dessous : 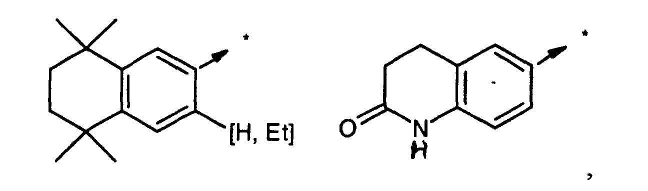 ou encore R3 représente un radical -CO-R5 ;
R4 représente H, alkyle, aralkyle carbocyclique ou hétérocyclique éventuellement situé sur le radical aryle ; ou alors le radical 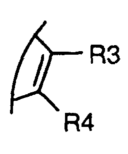 représente un radical de formule générale 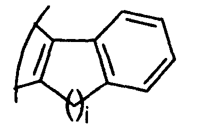 dans laquelle i représente un entier de 1 à 3 ;
R5 représente le radical N(R6)(R7) ;
R6 représente un radical (C₁-C₁₆)alkyle, cycloalkylalkyle, hydroxyalkyle, aryloxyalkyle, aralkyle carbocyclique ou hétérocyclique éventuellement substitué sur le groupe aryle, aralkoxyalkylke, arylhydroxyalkyle, alkoxyalkyle, alkylthioalkyle, alkényle, alkynyle, cyclohexényle, cyclohexénylalkyle, alkylthiohydroxyalkyle, cyanoalkyle, N-acétamidoalkyle, bis-arylalkyle éventuellement substitué sur les groupes aryle, di-arylalkyle éventuellement substitué sur les groupes aryle, morpholinoalkyle, pyrrolidinoalkyle, pipéridinoalkyle, N-alkylpyrrolidinoalkyle, oxopyrrolidinoalkyle, tétrahydrofurannylalkyle, N-benzylpyrrolidinoalkyle, N-alkylpipérazinylalkyle, N-benzylpipérazinylalkyle, N-benzylpipéridinylalkyle ou N-alkoxycarbonylpipéridinyle, ou R6 représente un radical (C₃-C₈)cycloalkyle éventuellement substitué par un radical choisi parmi le groupe composé du radical hydroxy et d'un radical alkyle,
   ou bien R6 représente l'un des radicaux représentés ci-dessous : 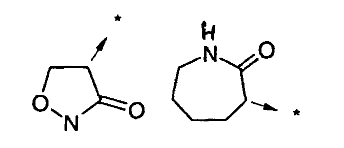 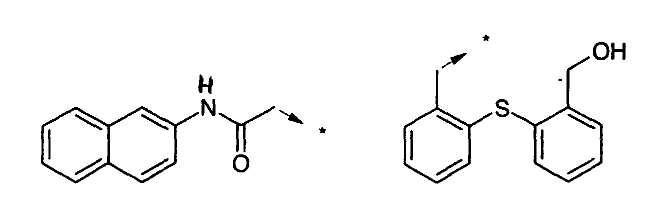
R7 représente H ou un radical alkyle, hydroxyalkyle, mono- ou di-aminoalkyle ou aralkyle ; ou encore le radical -N(R6)(R7) représente le radical de formule générale suivante : 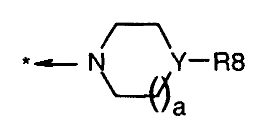 dans laquelle :
R8 représente H, alkyle, hydroxyalkyle, aryle carbocyclique ou hétérocyclique éventuellement substitué, aralkyle éventuellement substitué sur le groupe aryle, alkényle, alkoxyalkyle, cycloalkyle, cycloalkylalkyle, bis-arylalkyle, pipéridinyle, pyrrolidinyle, hydroxy, arylalkényle,
   ou R8 représente -X-(CH₂)_{b}-R9 ;
R9 représente H ou un radical alkyle, alkoxy, aryloxy, aryle carbocyclique ou hétérocyclique éventuellement substitué, morpholinyle, pyrrolidinyle, alkylamino ou N,N'-(alkyl)(aryl)amino ;
X représente CO, CO-NH ou SO₂ ;
Y représente CH ou N ;
a représente 1 ou 2 ;
b représente un entier de 0 à 6 ;
   ou le radical N(R6)(R7) représente un radical de formule générale 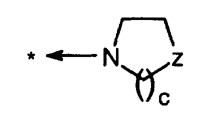 dans laquelle :
Z représente CH, O ou S ;
c représente un entier de 0 à 4 ;
   ou encore le radical N(R6)(R7) représente l'un des radicaux représentés ci-dessous : 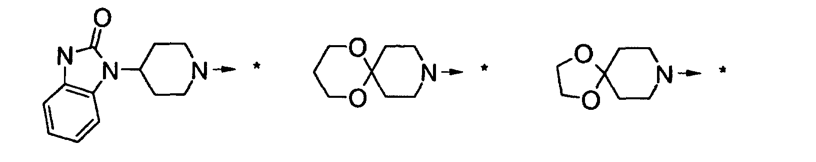 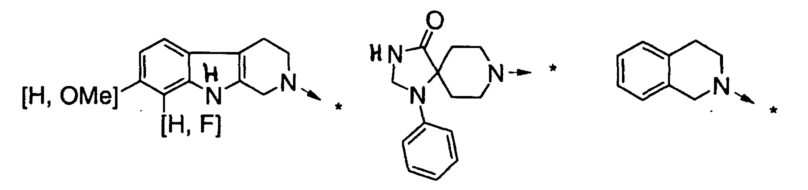 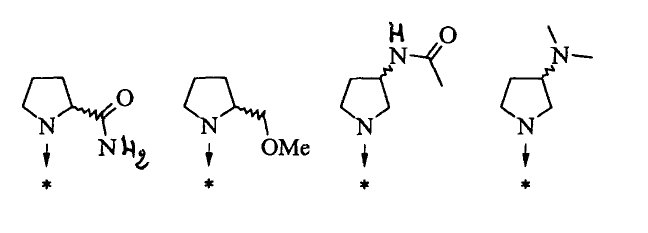 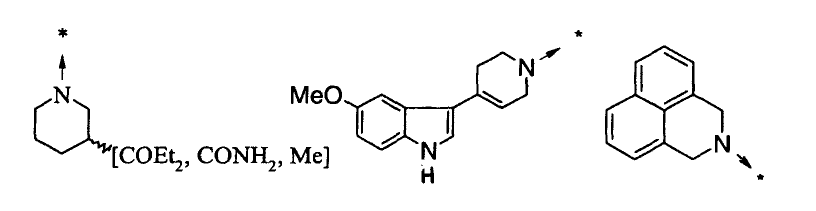 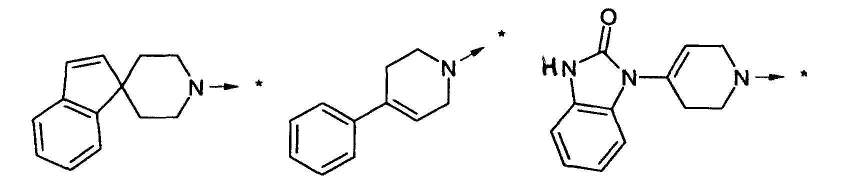
R10 représente un radical amino(C₂-C₇)alkylamino, ((aminoalkyl)aryl)alkylamino, ((aminoalkyl)cycloalkyl)alkylamino, pipérazinyle, homopipérazinyle,
   ou R10 représente le radical représenté ci-dessous : 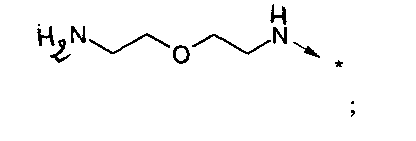
R11 représente H ;
R12 représente H ou un radical alkyle, (C₃-C₇)cycloalkyle, aralkyle carbocyclique ou hétérocyclique éventuellement substitué, propargyle, allyle, hydroxyalkyle, alkylthioalkyle, arylalkylalkoxyalkyle, arylalkylthioalkoxyalkyle ;
   et les sels desdits composés comme présentant une affinité pour les récepteurs de la somatostatine.

La présente invention concerne un composé caractérisé en ce qu'il correspond :
- à la formule 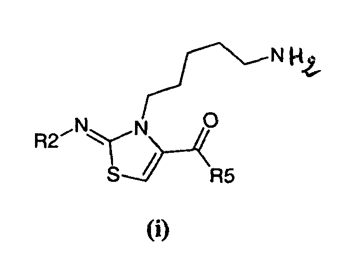 dans laquelle :
   - R2 représente le radical 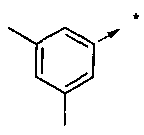 et R5 représente le radical 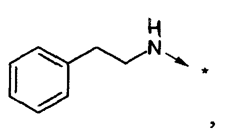
   - R2 représente le radical 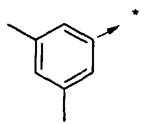 et R5 représente le radical 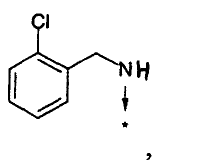
   - R2 représente le radical 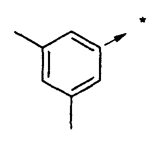 et R5 représente le radical 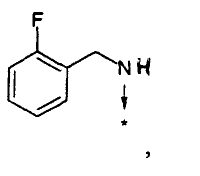
   - R2 représente le radical 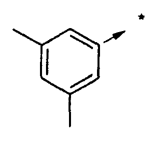 et R5 représente le radical 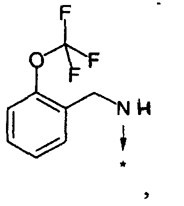
   - R2 représente le radical 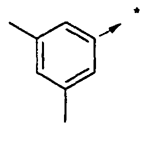 et R5 représente le radical 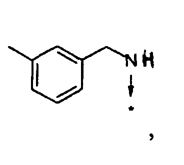
   - R2 représente le radical 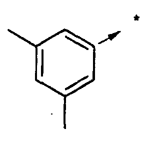 et R5 représente le radical 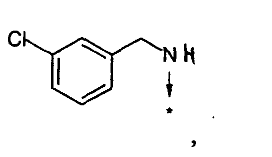
   - R2 représente le radical 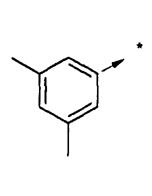 et R5 représente le radical 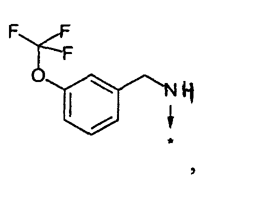
   - R2 représente le radical 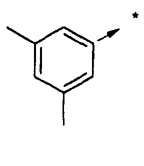 et R5 représente le radical 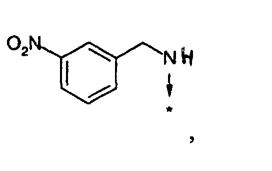
   - R2 représente le radical 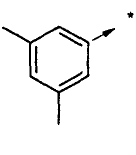 et R5 représente le radical 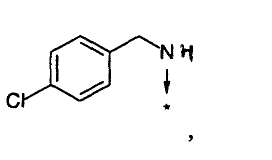
   - R2 représente le radical 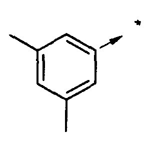 et R5 représente le radical 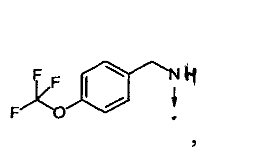
   - R2 représente le radical 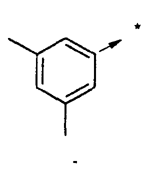 et R5 représente le radical 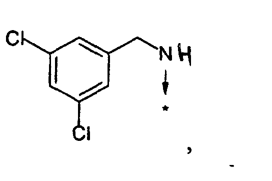
   - R2 représente le radical 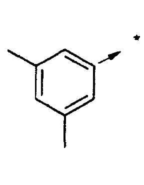 et R5 représente le radical 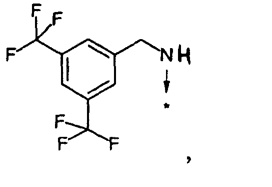
   - R2 représente le radical 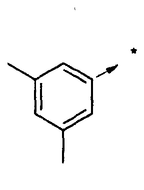 et R5 représente le radical 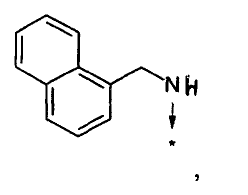
   - R2 représente le radical 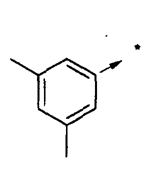 et R5 représente le radical 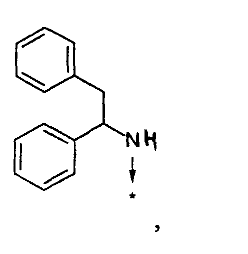
   - R2 représente le radical 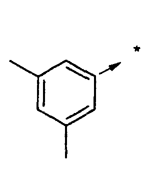 et R5 représente le radical 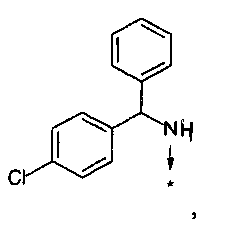
   - R2 représente le radical 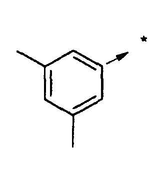 et R5 représente le radical 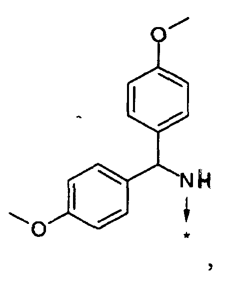
   - R2 représente le radical 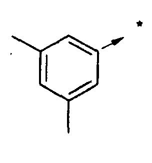 et R5 représente le radical 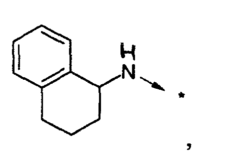
   - R2 représente le radical 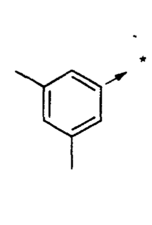 et R5 représente le radical 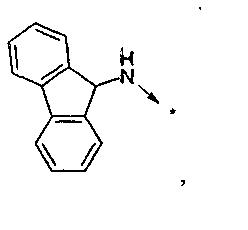
   - R2 représente le radical 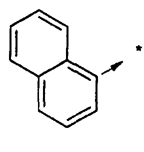 et R5 représente le radical 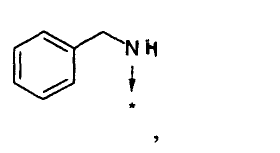
   - R2 représente le radical 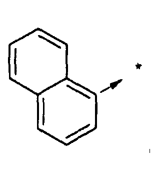 et R5 représente le radical 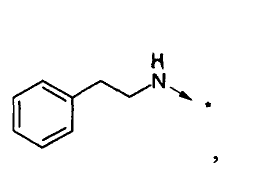
   - R2 représente le radical 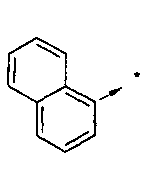 et R5 représente le radical 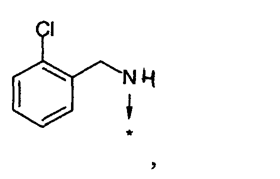
   - R2 représente le radical 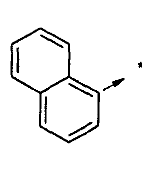 et R5 représente le radical 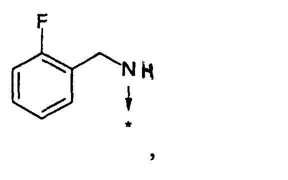
   - R2 représente le radical 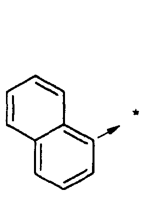 et R5 représente le radical 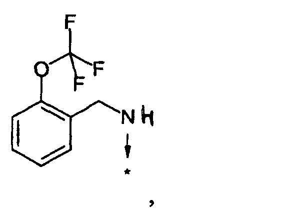
   - R2 représente le radical 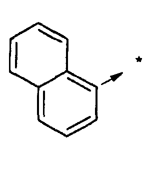 et R5 représente le radical 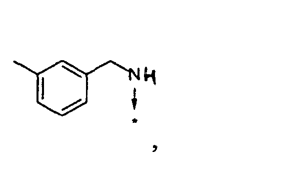
   - R2 représente le radical 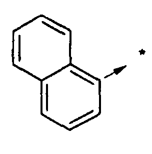 et R5 représente le radical 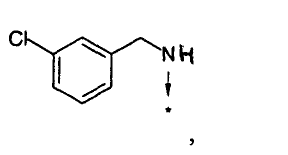
   - R2 représente le radical 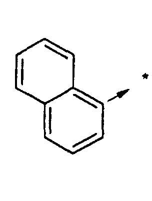 et R5 représente le radical 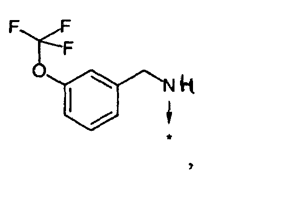
   - R2 représente le radical 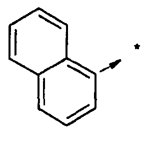 et R5 représente le radical 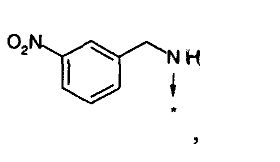
   - R2 représente le radical 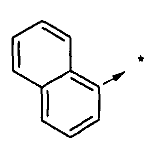 et R5 représente le radical 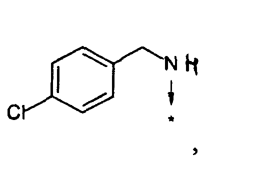
   - R2 représente le radical 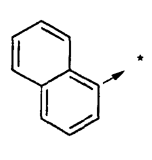 et R5 représente le radical 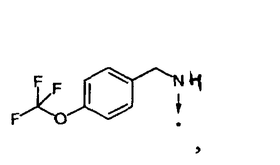
   - R2 représente le radical 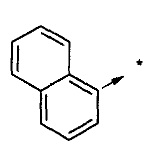 et R5 représente le radical 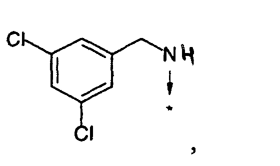
   - R2 représente le radical 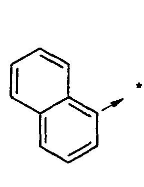 et R5 représente le radical 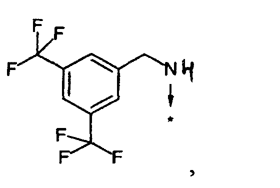
   - R2 représente le radical 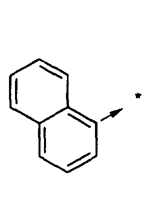 et R5 représente le radical 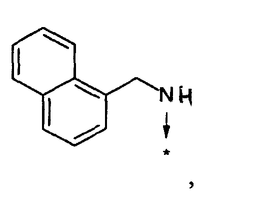
   - R2 représente le radical 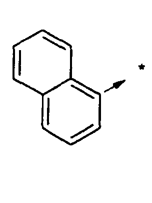 et R5 représente le radical 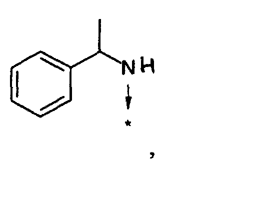
   - R2 représente le radical 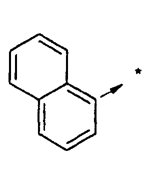 et R5 représente le radical 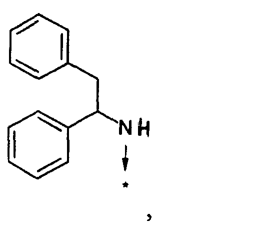
   - R2 représente le radical 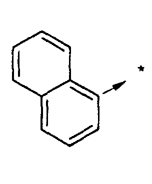 et R5 représente le radical 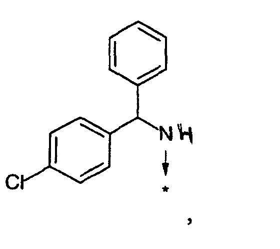
   - R2 représente le radical 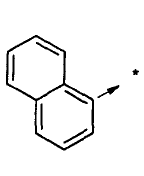 et R5 représente le radical 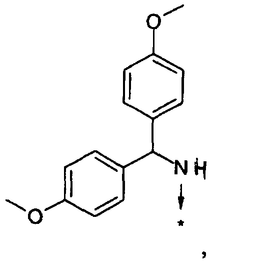
   - R2 représente le radical 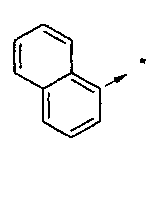 et R5 représente le radical 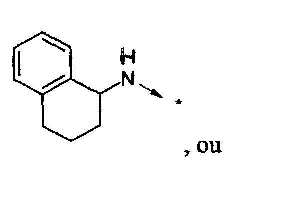 enfin
   - R2 représente le radical 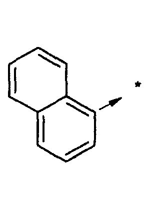 et R5 représente le radical 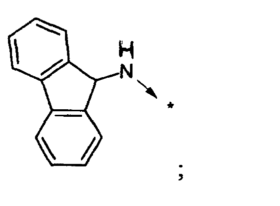
- ou à la formule 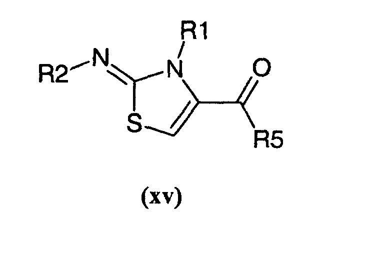 dans laquelle:
   - R1 représente 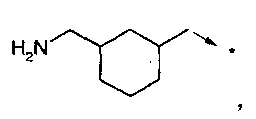 R2 représente 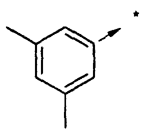 et R5 représente 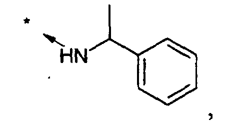
   - R1 représente 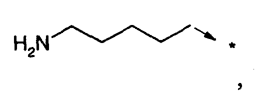 R2 représente 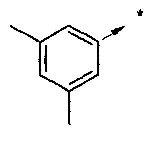 et R5 représente 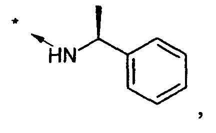
   - R1 représente 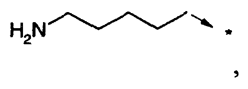 R2 représente 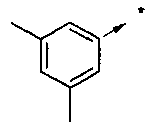 et R5 représente 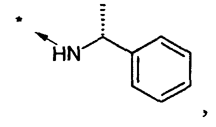
   - R1 représente 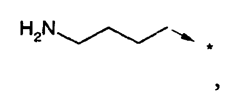 R2 représente 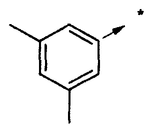 et R5 représente 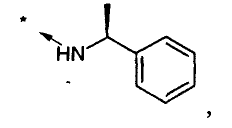
   - R1 représente 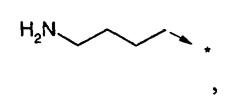 R2 représente 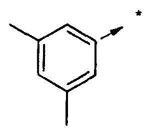 et R5 représente 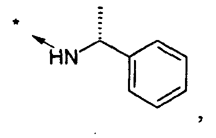
   - R1 représente 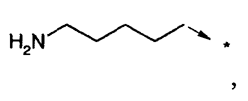 R2 représente 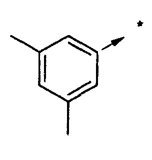 et R5 représente 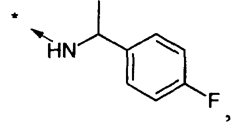
   - R1 représente 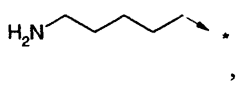 R2 représente 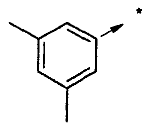 et R5 représente 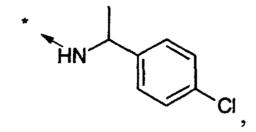
   - R1 représente 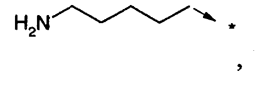 R2 représente 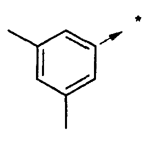 et R5 représente 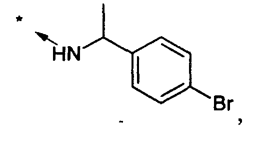
   - R1 représente 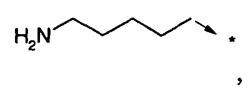 R2 représente 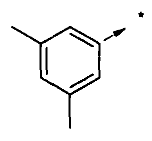 et R5 représente 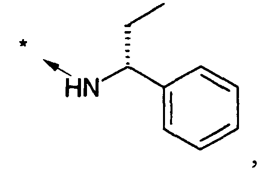 ou enfin
   - R1 représente 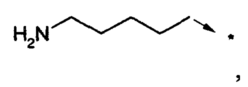 R2 représente 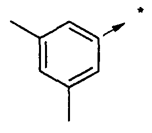 et R5 représente 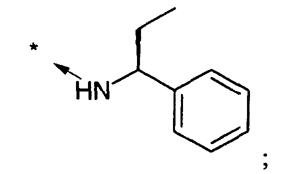 ou un sel d'un de ces composés.

De façon préférée, l'invention concerne un composé caractérisé en ce qu'il répond à la formule 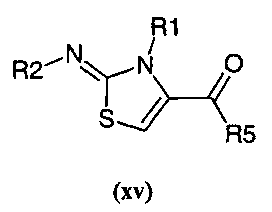 dans laquelle:
- R1 représente 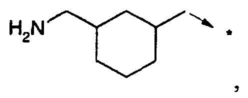 R2 représente 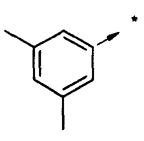 et R5 représente 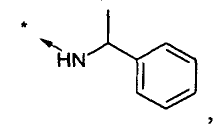
- R1 représente 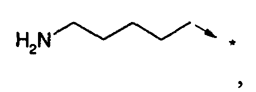 R2 représente 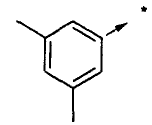 et R5 représente 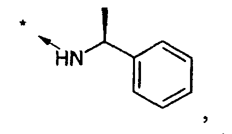
- R1 représente 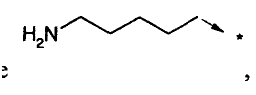 R2 représente 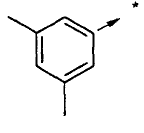 et R5 représente 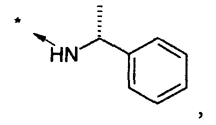
- R1 représente 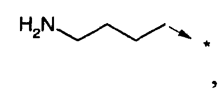 R2 représente 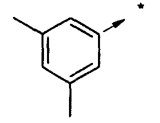 et R5 représente 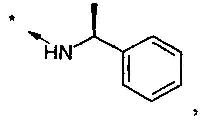
- R1 représente 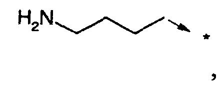 R2 représente 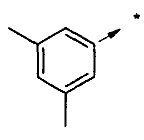 et R5 représente 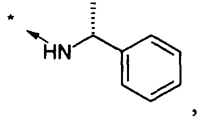
- R1 représente 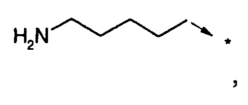 R2 représente 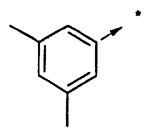 et R5 représente 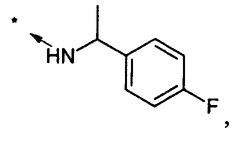
- R1 représente 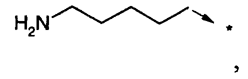 R2 représente 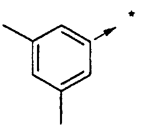 et R5 représente 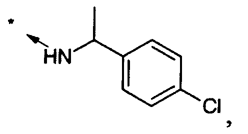
- R1 représente 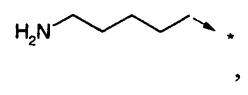 R2 représente 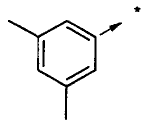 et R5 représente 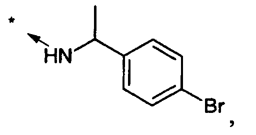
- R1 représente 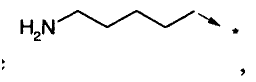 R2 représente 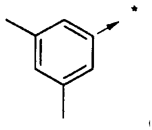 et R5 représente 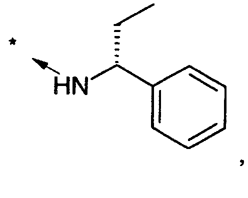 ou enfin
- R1 représente 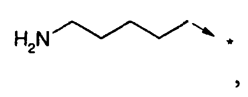
R2 représente 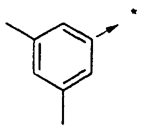 et R5 représente 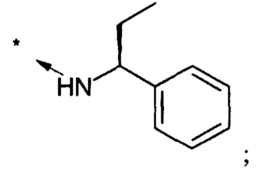 ou un sel d'un de ces composés.

En d'autres termes, seront préférés les composés décrits dans les exemples 1 à 49 ou les sels de ces composés. Seront encore plus particulièrement préférés les composés des exemples 40 à 49 ou leurs sels.

L'invention a également pour objet, à titre de médicaments, les composés de formules générales (i) et (xv) décrits précédemment ou leurs sels pharmaceutiquement acceptables. Elle concerne aussi des compositions pharmaceutiques comprenant lesdits composés ou leurs sels pharmaceutiquement acceptables, et leur utilisation pour la préparation d'un médicament destiné à traiter les états pathologiques ou les maladies dans lesquels un (ou plusieurs) des récepteurs de la somatostatine est (sont) impliqué(s).

En particulier, les composés de formules générales (i) et (xv) décrits précédemment ou leurs sels pharmaceutiquement acceptables pourront être utilisés pour la préparation d'un médicament destiné à traiter les états pathologiques ou les maladies choisis parmi le groupe composé des états pathologiques ou des maladies qui suivent : l'acromégalie, les adénomes hypophysaires, la maladie de Cushing, les gonadotrophinomes et les prolactinomes, les effets secondaires cataboliques des glucocorticoïdes, le diabète insulinodépendant, la rétinopathie diabétique, la néphropathie diabétique, le syndrome X, le phénomène de Dawn, l'angiopathie, l'angioplastie, l'hyperthyroïdie, le gigantisme, les tumeurs gastroentéropancréatiques endocriniennes dont le syndrome carcinoïde, le VIPome, l'insulinome, la nésidioblastose, l'hyperinsulinémie, le glucagonome, le gastrinome et le syndrome de Zollinger-Ellison, le GRFome ainsi que le saignement aigu des varices oesophagiennes, les ulcères, le reflux gastrooesophagien, le reflux gastroduodénal, la pancréatite, les fistules entérocutanées et pancréatiques mais aussi les diarrhées, les diarrhées réfractaires du syndrome d'immunodépression acquise, la diarrhée chronique sécrétoire, la diarrhée associée avec le syndrome de l'intestin irrité, les diarrhées induites par la chimiothérapie, les troubles liés au peptide libérateur de gastrine, les pathologies secondaires aux greffes intestinales, l'hypertension portale ainsi que les hémorragies des varices chez des malades avec cirrhose, l'hémorragie gastro-intestinale, l'hémorragie de l'ulcère gastroduodénale, le saignement des vaisseaux greffés, la maladie de Crohn, les scléroses systémiques, le dumping syndrome, le syndrome du petit intestin, l'hypotension, la sclérodermie et le carcinome thyroïdien médullaire, les maladies liées à l'hyperprolifération cellulaire comme les cancers et plus particulièrement le cancer du sein, le cancer de la prostate, le cancer thyroïdien ainsi que le cancer pancréatique et le cancer colorectal, les fibroses et plus particulièrement la fibrose du rein, la fibrose du foie, la fibrose du poumon, la fibrose de la peau, également la fibrose du système nerveux central ainsi que celle du nez et la fibrose induite par la chimiothérapie, et, dans d'autres domaines thérapeutiques, les céphalées y compris les céphalées associées aux tumeurs hypophysaires, les douleurs, les désordres inflammatoires comme l'arthrite, les accès de panique, la chimiothérapie, la cicatrisation des plaies, l'insuffisance rénale résultant d'un retard de croissance, l'hyperlipidémie, l'obésité et le retard de croissance lié à l'obésité, le retard de croissance utérin, la dysplasie du squelette, le syndrome de Noonan, le syndrome d'apnée du sommeil, la maladie de Graves, la maladie polykystique des ovaires, les pseudokystes pancréatiques et ascites, la leucémie, le méningiome, la cachexie cancéreuse, l'inhibition des H pylori, le psoriasis, le rejet chronique des allogreffes ainsi que la maladie d'Alzheimer et enfin l'ostéoporose.

De préférence, les composés de formules générales (i) et (xv) décrits précédemment ou leurs sels pharmaceutiquement acceptables pourront être utilisés pour la préparation d'un médicament destinés à traiter les états pathologiques ou les maladies choisis parmi le groupe composé des états pathologiques ou des maladies qui suivent : l'acromégalie, les adénomes hypophysaires ou les tumeurs gastroentéropancréatiques endocriniennes dont le syndrome carcinoïde, et les saignements gastro-intestinaux.

Par sel pharmaceutiquement acceptable, on entend notamment des sels d'addition d'acides inorganiques tels que chlorhydrate, sulfate, phosphate, diphosphate, bromhydrate et nitrate ou d'acides organiques tels que acétate, maléate, fumarate, tartrate, succinate, citrate, lactate, méthane sulfonate, p-toluènesulfonate, pamoate, oxalate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Pharmaceutical salts", *J. Pharm. Sci*. **66**:1 (1977).

La composition pharmaceutique peut être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire. Les suspensions comprendront en particulier les suspensions de microparticules à libération prolongée chargées en principe actif (notamment des microparticules en polylactide-co-glycolide ou PLGA - cf. par exemple les brevets US 3,773,919, EP 52 510 ou EP 58 481 ou la demande de brevet PCT WO 98/47489), lesquelles permettent l'administration d'une dose journalière déterminée sur une période de plusieurs jours à plusieurs semaines.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

Ces composés peuvent être préparés selon les méthodes décrites ci-après.

### PREPARATION DES COMPOSES DE L'INVENTION

### I) Préparation d'α-bromocétones

### PREMIERE METHODE

Cette méthode s'inspire des protocoles décrits dans les publications suivantes : Macholan, L.; Skursky, L. *Chem. Listy* **1955**, *49*, 1385-1388 ; Bestman, H.J. ; Seng, F. *Chem. Ber*. **1963**, *96*, 465-469 ; Jones, R.G. ; Kornfeld, E.C. ; McLaughlin, K.C. *J. Am. Chem. Soc.* **1950,** *72*, 4526-4529 ; Nimgirawath, S. ; Ritchie, E. ; Taylor, W.C. *Aust. J. Chem.* **1973**, *26*, 183-193).

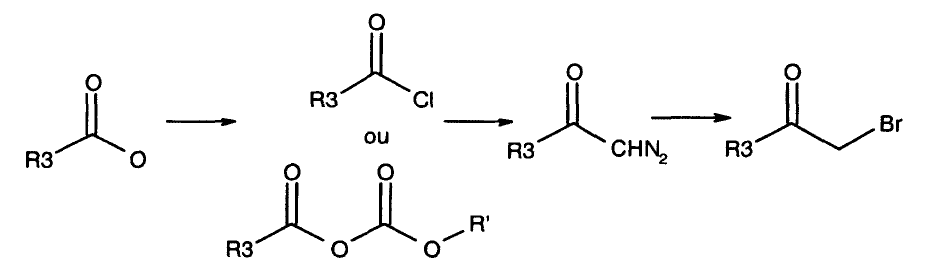

Un acide carboxylique est tout d'abord converti en un chlorure d'acide en utilisant du chlorure d'oxalyle ou de thionyle, ou en l'activant sous forme d'un anhydride à l'aide d'un chloroformiate d'alkyle (par exemple un chloroformiate d'isobutyle, cf. Krantz, A. ; Copp, L.J. *Biochemistry* **1991,** *30,* 4678-4687 ; ou un chloroformiate d'éthyle, cf. Podlech, J. ; Seebach, D. *Liebigs Ann.* **1995,** 1217-1228) en présence d'une base (triéthylamine ou N-méthylmorpholine).

Le groupe carboxyle activé est ensuite transformé en diazocétone à l'aide de diazométhane en solution éthérée ou d'une solution commerciale de triméthylsilyldiazométhane (Aoyama, T. ; Shiori, T. *Chem. Pharm. Bull.* **1981,** *29*, 3249-3255) dans un solvant aprotique comme le diéthyléther, le tétrahydrofuranne (THF) ou l'acétonitrile.

La bromation est ensuite effectuée en utilisant un agent bromant comme l'acide hydrobromique dans l'acide acétique, l'acide hydrobromique aqueux dans le diéthyléther ou le dichlorométhane.

### Préparation 1

### 2-(4-bromo-3-oxobutyl)-1H-isoindole-1,3(2H)-dione (C₁₂H₁₀BrNO₃, MM = 296,12) :

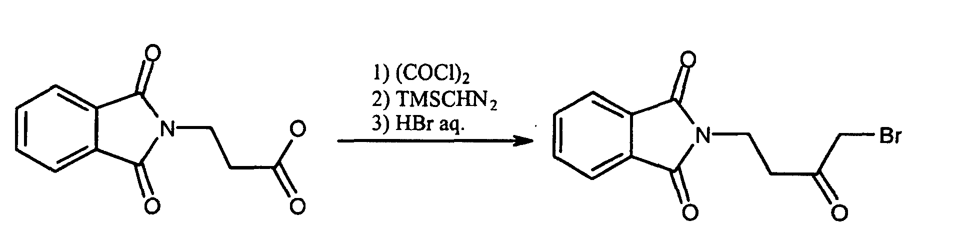

Du chlorure d'oxalyle (5,8 ml ; 66,7 mmol) est ajouté à Pht-β-Ala-OH (9,96g; 44,5 mmol) dissous dans du dichlorométhane (120ml) et 3 gouttes de diméthylformamide (DMF). Le mélange est agité pendant 3 heures à température ambiante. Après élimination du solvant, le solide blanc est repris dans un mélange 1:1 de tétrahydrofuranne anhydre et d'acétonitrile (200 ml) puis 49 ml de solution de (triméthylsilyl)diazaméthane 2M dans l'hexane (97,9 mmol) sont ajoutés goutte à goutte à 0 °C. Les solvants sont éliminés après une nuit sous agitation à 0 °C. Le solide jaune pâle est alors dissous dans du dichlorométhane (60 ml) et 12 ml d'acide hydrobromique acqueux (48%) sont ajoutés goutte à goutte à 0 °C. Le mélange est agité jusqu'à ce que la température remonte à 15 °C et 50 ml de solution saturée en bicarbonate de sodium sont ajoutés. La phase organique est lavée avec de la saumure puis séchée sur du sulfate de sodium. La cristallisation dans du diéthyléther permet d'obtenir un solide blanc (11,39 g ; rendement = 86%).
RMN ¹H (DMSO D6, 100 MHz, δ) : 7,83 (s, 4H) ; 4,36 (s, 2H, CH₂Br) ; 3,8 (t, 2H, J = 7,1 Hz, NCH₂) ; 2,98 (t, 2H, J = 6,9 Hz, CH₂CO).

### Préparations 2-11

Les composés suivants ont été préparés de façon analogue à la procédure décrite dans la Préparation 1 :

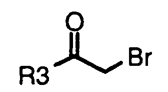

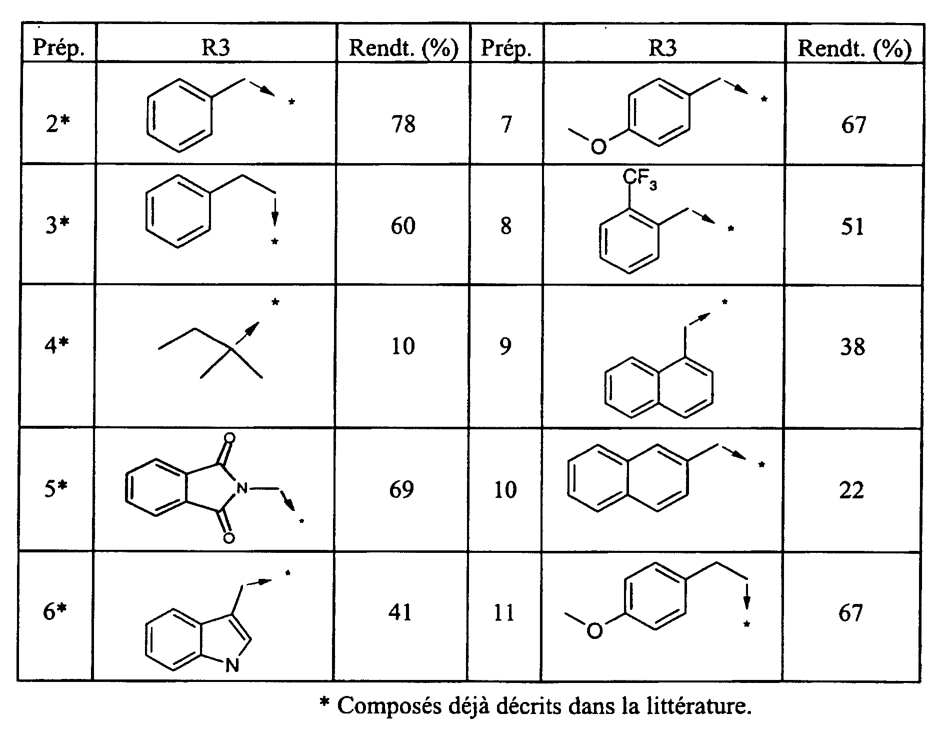

### DEUXIEME METHODE

Le produit de départ est une arylméthylcétone ou une hétéroarylméthylcétone.

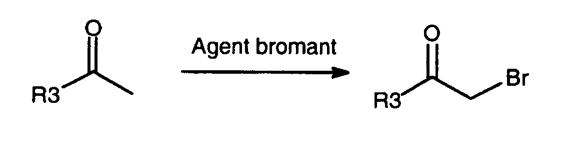

L'arylméthylcétone ou l'hétéroarylméthylcétone de départ est convertie en l'α-bromocétone correspondante en utilisant différents agents bromants :
- CuBr₂ (King, L.C. ; Ostrum, G.K. *J. Org. Chem.* **1964,** *29,* 3459-3461) chauffé dans de l'acétate d'éthyle ou du dioxane ;
- du N-bromosuccinimide dans CCl₄ ou de l'acétonitrile aqueux (Morton, H.E.; Leanna, M.R. *Tetrahedron Lett.* **1993**, *34*, 4481-4484) ;
- du brome dans de l'acide acétique glacial ou de l'acide sulfurique ;
- du tribromure de phényltriméthylammonium (Sanchez, J. P. ; Parcell, R. P. *J. Heterocyclic Chem,* **1988**, *25*, 469-474) à 20-80 °C dans un solvant aprotique comme le THF ou du tribromure de tétrabutylammonium (Kajigaeshi, S.; Kakinami, T.; Okamoto, T.; Fujisaki, S. *Bull. Chem. Soc. Jpn*. **1987,** *60,* 1159-1160) dans un mélange dichlorométhane/méthanol à température ambiante ;
- agent bromant sur un support polymère comme du perbromure sur une résine Amberlyst A-26, poly(perbromure d'hydrobromure de vinylpyridinium) (Frechet, J. M. J. ; Farrall, M. J. *J. Macromol. Sci. Chem.* **1977,** 507-514) dans un solvant protique comme le méthanol à environ 20-35 °C pendant environ 2-10 h.

### Préparation 12

### 1-(1-benzofuran-2-yl)-2-bromo-1-éthanone (C₁₀H₇BrO₂, MM = 239,06) :

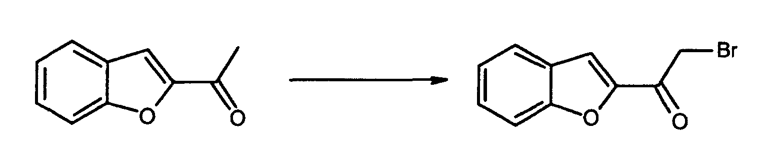

A une solution de (benzofuran-2-yl)méthylcétone (2 g ; 12,5 mmol) dans du méthanol (40 ml) est ajouté un polymère de perbromure d'hydrobromure de pyridine (8,75 g ; 17,5 mmol ; 1,4 équivalent). Le mélange résultant est agité à température ambiante pendant 7 heures et la réaction est arrêtée par filtration. Le méthanol est éliminé sous pression réduite et une addition supplémentaire de diéthyléther permet la cristallisation du produit attendu (3,6 g ; rendement = 60%).
RMN ¹H (DMSO D6, 100 MHz, δ) : 8,09 (s, 1H) ; 7,98 (d, 1H, J = 6,6 Hz) ; 7,75 (d, 1H, J = 8,4 Hz) ; 7,58 (t, 1H, J = 8,4 Hz) ; 7,4 (t, 1H, J = 7 Hz) ; 4,83 (s, 2H, CH₂Br).

### Préparations 13-18

Les composés suivants ont été préparés de façon analogue à la procédure décrite dans la Préparation 12 :

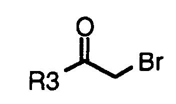

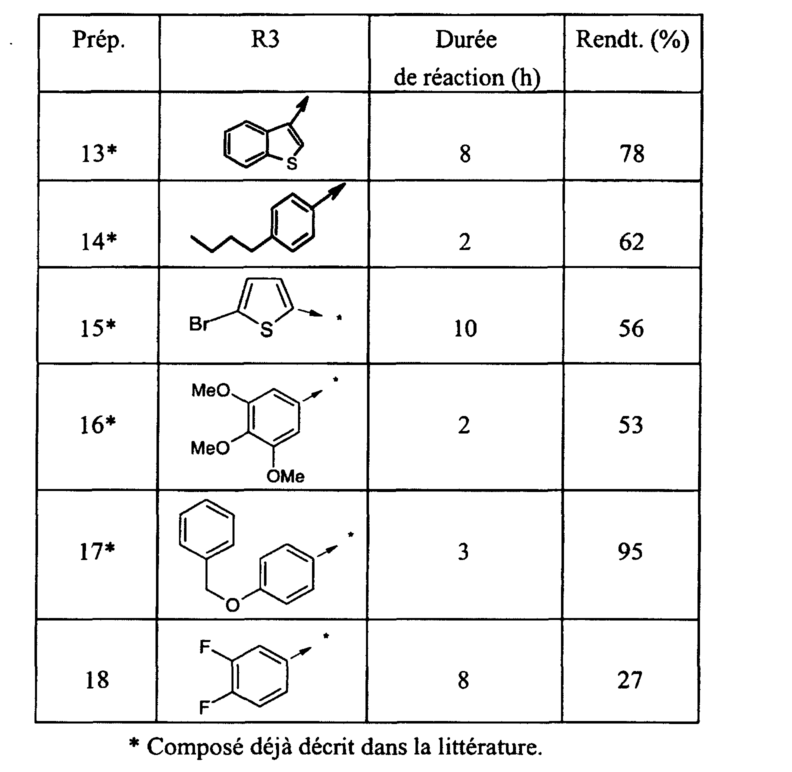

### II) Synthèse de 2-arylimino-2,3-dihydrothiazoles via synthèse sur phase solide

### Préparation de la résine p-nitrophénylcarbonate de Wang

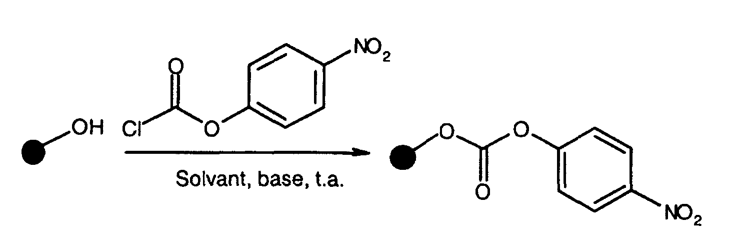

Cette résine a été préparée à partir de résine de Wang, acquise auprès de Bachem ou Novabiochem avec une charge supérieure à 0,89 mmol/g, par une procédure générale bien décrite (cf. Bunin, B.A. *The Combinatorial Index,* Academic Press, **1998,** p. 62-63 ; Dressman, B.A. ; Spangle, L.A. ; Kaldor, S.W. *Tetrahedron Lett.* **1996,** *37,* 937-940; Hauske, J.R. ; Dorff, P. *Tetrahedron Lett.* **1995,** *36,* 1589-1592; Cao, J. ; Cuny, G.D. ; Hauske, J.R. *Molecular Diversity* **1998,** *3,* 173-179): de la N-méthylmorpholine ou de la pyridine comme base et du 4-nitrophénylchloroformiate sont successivement ajoutés à une résine de Wang pré-gonflée dans du dichlorométhane (DCM) ou du tétrahydrofuranne (THF) à température ambiante. Le mélange est agité pendant la nuit. La résine est alors lavée successivement avec du THF, du diéthyléther et du DCM puis séchée sous pression réduite à 50 °C pendant une nuit.

### METHODE A

### Préparation de diamines symétriques monoprotégées

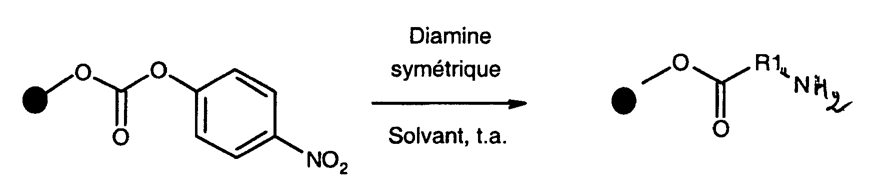

Procédure générale : comme déjà décrit dans la littérature (Dixit, D.M. ; Leznoff, C.C. *J. C. S. Chem. Comm.* **1977,** 798-799 ; Dixit, D.M. ; Leznoff, C.C. *Israel J. Chem*. **1978,** *17*, 248-252 ; Kaljuste K. ; Unden, A. *Tetrahedron Lett.* **1995,** *36,* 9211-9214 ; Munson, M.C. ; Cook, A.W. ; Josey, J.A. ; Rao, C. *Tetrahedron Lett.* **1998,** *39,* 7223-7226), une résine p-nitrophénylcarbonate de Wang est traitée avec un large excès de diamine symétrique (10-20 équivalents), dans un solvant aprotique comme le DCM ou le DMF, pour donner une résine diamine monoprotégée après agitation pendant la nuit.

### Préparation de résines thiourées

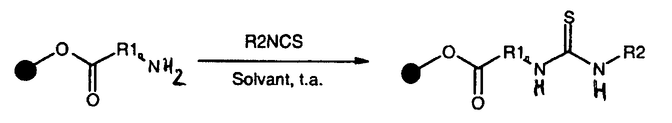

Procédure générale : des isothiocyanates aromatiques et hétéroaromatiques (5-10 équivalents) sont ajoutés (Smith, J. ; Liras, J.L. ; Schneider, S.E. ; Anslyn, E.V. *J.* *Org. Chem*. **1996,** *61,* 8811-8818) à des diamines symétriques monoprotégées dans un solvant comme le DCM ou le DMF agité pendant la nuit à température ambiante. Lavée successivement avec du DMF et du DCM, la résine thiourée est isolée puis séchée sous pression réduite à 50 °C pendant une nuit.

### Préparation 19

### Résine de Wang carbamate de (phénylaminothioyl)éthyle

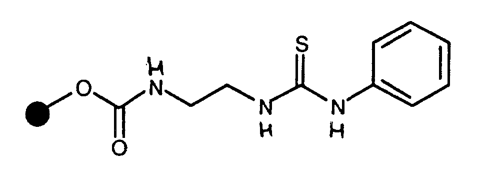

A une résine de Wang N-carbamate d'éthylène diamine (2 g ; 1,72 mmol ; 0,86 mmol/g) gonflée dans du DCM (50 ml) est ajouté du phénylisothiocyanate (1 ml ; 8,5 mmol ; 5 éq.). Après agitation une nuit à température ambiante, la résine est lavée successivement avec du DMF (5 x 20 ml) et du DCM (5 x 20 ml). La réussite du couplage est suivie à l'aide du test ninhydrine de Kaiser (Kaiser, E. ; Colescott, R.L. ; Bossinger, C.D. ; Cook, P.I. *Anal. Biochem*. **1970,** *34,* 595-598). Une résine jaune pâle (1,79 g) est obtenue avec une charge de 0,648 mmol/g calculée à partir de l'analyse élémentaire du soufre.

### METHODE B

### Préparation de résines de Wang carbamates à partir d'aminoalkylanilines

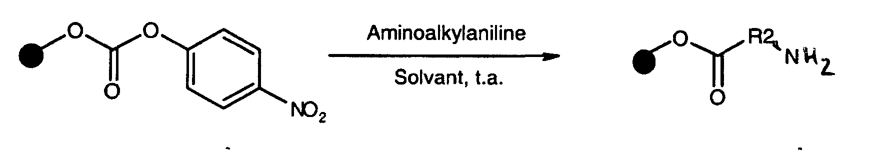

Procédure générale : comme déjà décrit (Hulme, C. ; Peng, J. ; Morton, G. ; Salvino, J.M. ; Herpin, T. ; Labaudiniere, R. *Tetrahedron Lett.* **1998**, *39*, 7227-7230), une résine de Wang p-nitrophénylcarbonate est traitée avec un excès d'aminoalkylaniline (5-10 éq.) dans du DCM ou du DMF et agitée à température ambiante durant une nuit. La résine est lavée successivement avec du DMF, du méthanol et du DCM puis séchée une nuit sous pression réduite à 50 °C.

### Préparation 20

### Résine de Wang carbamate de 4-aminophényléthyle

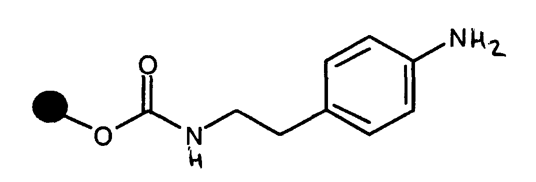

A une résine de Wang p-nitrophénylcarbonate (4,05 g ; 3,47 mmol ; charge de 0,857 mmol/g) pré-gonflée dans 50 ml de DMF anhydre est ajoutée une solution de 2-(4-aminophényl)éthylamine (2,48 g ; 17,3 mmol ; 5 éq.) dans 30 ml de DMF anhydre. Le mélange est agité à température ambiante durant une nuit et filtré. La résine est lavée successivement avec du DMF (10 x 30 ml), du méthanol (5 x 30 ml) et du DCM (5 x 30 ml). 3,7 g de résine jaune (charge de 0,8 mmol/g calculée à partir de l'analyse élémentaire de l'azote), donnant un test ninhydrine de Kaiser positif, sont isolés après séchage une nuit sous pression réduite à 50 °C.

### Préparation de résines thiourées avec des isothiocyanates aliphatiques

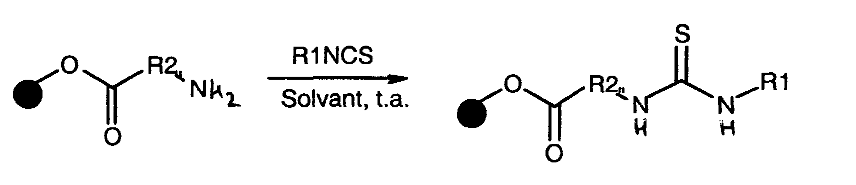

Procédure générale : des isothiocyanates aliphatiques (5-10 équivalents) sont ajoutés à une résine aminoalkylaniline dans un solvant comme le DCM ou le DMF et agités à température ambiante durant une nuit. Lavée successivement avec du DMF et du DCM, la résine thiourée est isolée et séchée sous pression réduite à 50 °C pendant une nuit.

### Préparation 21

### Résine de Wang carbamate de 4-{[(phényléthylamino)carbothioyl]amino}-phényléthyle

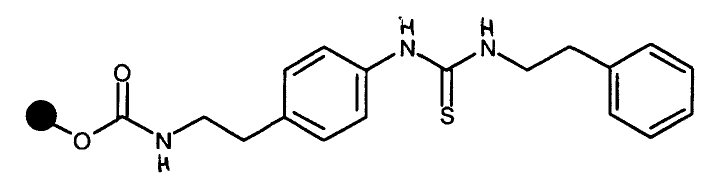

10 ml de DMF anhydre et du phényléthylisothiocyanate (624 µl, 4 mmol, 10 éq.) sont ajoutés sous atmosphère d'argon à la résine précédemment décrite (0,5 g ; 0,4 mmol ; charge de 0,8 mmol/g). L'agitation durant une nuit à température ambiante donne un test ninhydrine de Kaiser négatif. La résine est alors successivement lavée avec du DMF (5 x 20 ml) et du DCM (5 x 20 ml). Un séchage sous pression réduite à 50 °C donne 488 mg de résine avec une charge de 0,629 mmol/g calculée à partir de l'analyse élémentaire du soufre.

### METHODE C

### Synthèse de 2-arylimino-1,3-thiazole-4(3H)-carboxamides

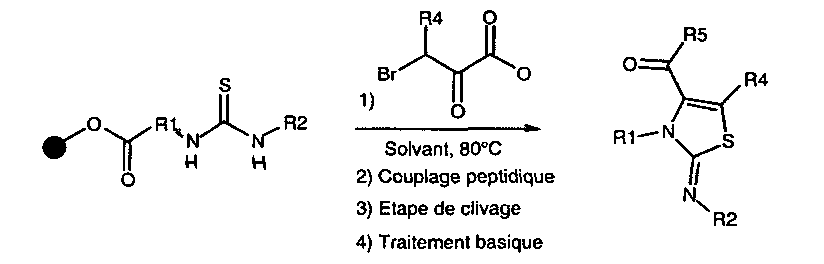

Procédure générale : une étape de cyclisation régiosélective à l'aide d'acide α-bromopyruvique (2-5 éq.) est effectuée à partir de la résine thiourée préparée dans la méthode A dans des solvants aprotiques comme le dioxane ou le DMF à 80 °C durant 2-3 heures. La résine est alors successivement lavée avec du DMF, du méthanol et du DCM puis séchée sous pression réduite. Le couplage peptidique (Knorr, R. ; Trzeciak, A. ; Bannwarth, W. ; Gillessen, D. *Tetrahedron Lett.* **1989,** *30,* 1927-1930) a lieu dans le DMF à température ambiante pendant 1-24 heures avec différents agents de couplage classiques (4-5 éq.) comme le dicyclohexylcarbodiimide (DCC), le diisopropylcarbodiimide (DIC), un mélange DIC/N-hydroxybenzotriazole (HOBt), l'hexafluorophosphate de benzotriazolyloxytris(diméthylamino)phosphonium (PyBOP), l'hexafluorophosphate de 2-(1*H*-benzotriazol-1-yl)-1,1,3,3-tétraméthyluronium (HBTU) ou le tétrafluoroborate de 2-(1*H*-benzotriazol-1-yl)-1,1,3,3-tétraméthyluronium (TBTU) et des composés aminés (4-5 éq.). La résine 2-arylimino-1,3-thiazole-4(3*H*)-carboxamide est clivée par traitement dans des conditions acides (DCM/acide trifluoroacétique à 50%) pendant 1-2 heures puis rinçage avec du DCM. Le solvant est évaporé et la base libre isolée après traitement dans des conditions basiques (solution saturée en hydrogénocarbonate de sodium), extraction avec du DCM ou élution avec du méthanol dans une cartouche d'alumine basique (500 mg, Interchim).

### Préparation 22

### 3-(4-aminobutyl)-N-benzhydryl-2-[(4-bromophényl)imino]-1,3-thiazole-4(3H)-carboxamide (C₂₇H₂₇BrN₄OS, MM = 535,51) :

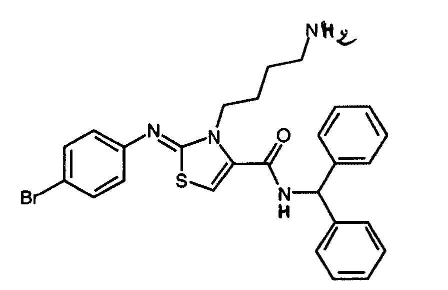

50 mg (27,5 µmol, charge de 0,55 mmol/g) de résine acide carboxylique est activée pendant 15 minutes avec 14,8 mg (0,11 mmol, 4 éq.) de N-hydroxybenzotriazole et 35,3 mg (0,11 mmol, 4 éq.) de TBTU dans 800 µl de DMF anhydre. 20,7 mg (0,11 mmol, 4 éq.) d'aminodiphénylméthane dissous dans 200 µl de DMF anhydre sont alors ajoutés et la résine est filtrée après agitation durant une nuit à température ambiante. Un lavage séquentiel avec du DMF (5 x 1 ml), du méthanol (5 x 1 ml) et du DCM (5 x 1 ml) donne une résine qui est traitée pendant une heure et demie dans des conditions acides (DCM/acide trifluoroacétique à 50 %). La résine est rincée avec du DCM (5 x 1 ml) et le filtrat évaporé sous pression réduite. Le résidu, repris dans du méthanol, est élué dans une cartouche d'alumine basique (500 mg, Interchim) pour donner un solide jaune pâle (8,2 mg ; rendement de 55,7 % ; pureté UV de 94 % à 220 nm).
RMN ¹H (DMSO D6, 100 MHz, δ): 9,6 (d ; 1H ; J = 8,6Hz ; NH) ; 7,49 (d ; 2H ; J = 8,6 Hz) ; 7,35 (s ; 10H) ; 6,92 (s ; 1H ; H azole) ; 6,91 (d ; 2H ; J = 8,5 Hz) ; 6,27 (d ; 1H ; J = 8,5 Hz ; NHCH) ; 4,02 (m ; 2H ; NCH₂) ; 3,45 (m large ; 2H+2H ; NH₂ et NCH₂) ; 1,55-1,24 (m large ; 4H). SM/CL : m/z = 535 (M+H).

### METHODE D

### Synthèse de 2-arylimino-1,3-thiazole-4(3H)-carboxamides

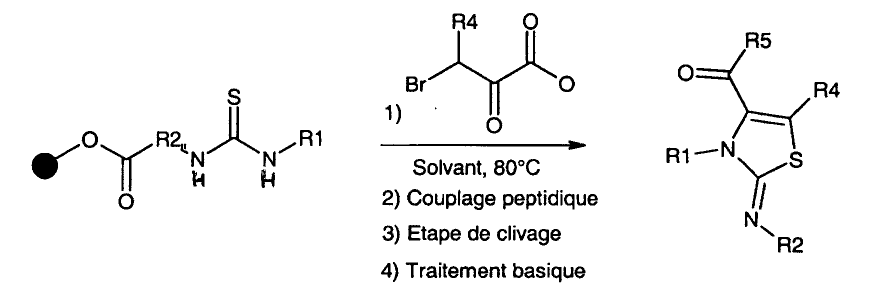

Procédure générale : une étape de cyclisation régiosélective à l'aide d'acide α-bromopyruvique (2-5 éq.) est effectuée à partir de la résine thiourée préparée dans la méthode B dans des solvants aprotiques comme le dioxane ou le DMF à 80 °C durant 2-3 heures. La résine est alors successivement lavée avec du DMF, du méthanol et du DCM puis séchée sous pression réduite. Le couplage peptidique (Knorr, R. ; Trzeciak, A. ; Bannwarth, W. ; Gillessen, D. *Tetrahedron Lett.* **1989,** *30,* 1927-1930) a lieu dans le DMF à température ambiante pendant 1-24 heures avec différents agents de couplage classiques (4-5 éq.) comme le dicyclohexylcarbodiimide (DCC), le diisopropylcarbodiimide (DIC), un mélange DIC/N-hydroxybenzotriazole (HOBt), l'hexafluorophosphate de benzotriazolyloxytris(diméthylamino)phosphonium (PyBOP), l'hexafluorophosphate de 2-(1*H*-benzotriazol-1-yl)-1,1,3,3-tétraméthyluronium (HBTU) ou le tétrafluoroborate de 2-(1*H*-benzotriazol-1-yl)-1,1,3,3-tétraméthyluronium (TBTU) et des composés aminés (4-5 éq.). La résine 2-arylimino-1,3-thiazole-4(3*H*)-carboxamide est clivée par traitement dans des conditions acides (DCM/acide trifluoroacétique à 50%) pendant 1-2 heures puis rinçage avec du DCM. Le solvant est évaporé et la base libre isolée après traitement dans des conditions basiques (solution saturée en hydrogénocarbonate de sodium) suivi d'une extraction avec du DCM ou élution avec du méthanol dans une cartouche d'alumine basique (500 mg, Interchim).

### Préparation 23

### (2Z)-2-{[4-(2-aminoéthyl)phényl]imino}-N-(4-chlorobenzyl)-3-(2-phényléthyl)-2,3-dihydro-1,3-thiazole-4-carboxamide (C₂₇H₂₇ClN₄OS, MM = 491,05) :

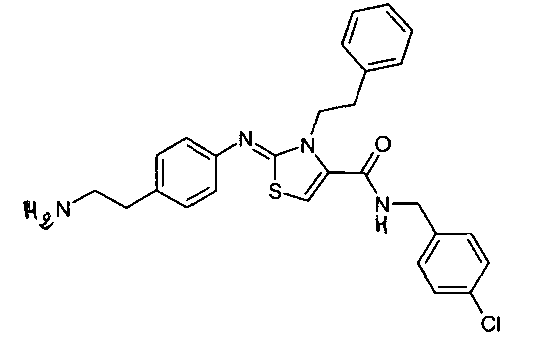

A 200 mg (190 µmol, charge de 0,946 mmol/g) de résine aminée (voir préparation 20) est ajouté du phényléthylisothiocyanate (310 mg; 1,9 mmol ; 10 éq.) dans 3 ml de diméthylformamide. L'agitation durant une nuit à température ambiante donne un test ninhydrine de Kaiser négatif. La résine est alors successivement lavée avec du DMF (5 x 3 ml) et du DCM (5 x 3 ml) puis séchée sous vide pendant une heure avant d'ajouter l'acide bromopyruvique (63,4 mg ; 380 µmol ; 2 éq.) préalablement dilué dans 3 ml de diméthylformamide. Le mélange est agitée pendant 2,5 heures à 80°C. Filtrer et laver la résine au DMF (5 x 3 ml), méthanol (3 x 3 ml) puis DCM (5 x 3 ml). La résine acide carboxylique est préactivée pendant 1 heure avec 244 mg (0,76 mmol ; 4 éq.) de TBTU dilué dans 2 ml de DMF anhydre. 110 mg (0,76 mmol ; 4 éq.) de 4-chlorobenzylamine dissous dans 1 ml de DMF anhydre sont alors ajoutés et la résine est filtrée après une nuit d'agitation à température ambiante. Un lavage séquentiel avec du DMF (5 x 3 ml), du méthanol (3 x 3 ml) et du DCM (3 x 3 ml) donne une résine qui est traitée pendant une heure et demie dans des conditions acides (DCM/acide trifluoroacétique à 50 %). La résine est rincée avec du DCM (5 x 1 ml) et le filtrat évaporé sous pression réduite. Le résidu, repris dans du DCM, est neutralisé avec une solution saturée d'hydrogénocarbonate de sodium- pour donner après évaporation un solide (38,2 mg ; rendement de 41% ; pureté UV de 90% à 210 nm).
RMN ¹H (DMSO D6, 400 MHz, δ) : 9,1 (m, 1H) ; 7,39 (d, 2H, J = 8,4 Hz) ; 7,33 (d, 2H, J = 8,4 Hz) ; 7,25 (q, 2H, J = 6,8 Hz) ; 7,19 (q, 1 H, J = 7,2 Hz) ; 7,11 (m, 4H) ; 6,8 (d, 2H, J = 8 Hz) ; 6,75 (s, 1H, *H* azole) ; 4,34 (d, 2H, J = 6 Hz) ; 4,27 (t, 2H, J = 6,8 Hz) ; 3,14 (m, 1H) ; 2,89 (t, 2H, J = 6,8 Hz) ; 2,73 (t, 1H, J = 7,2 Hz) ; 2,62 (m, 2H). SM/CL : m/z = 491,24 (M+H)⁺.

### EXEMPLES

Ci-après sont repris dans des tableaux des exemples obtenus selon les méthodes A, B, C et D décrites ci-dessus. Ces exemples sont présentés pour illustrer les procédures ci-dessus et ne doivent en aucun cas être considérés comme une limite à la portée de l'invention.

Les composés obtenus ont été caractérisés grâce à leur temps de rétention (tr) et à la spectrométrie de masse (M+H)⁺.

Les chromatogrammes sont obtenus à partir d'un appareil de chromatographie liquide haute performance (Hewlett-Packard 1100) équipé d'un détecteur UV à balayage. Les conditions suivantes ont été employées pour les mesures des temps de rétention par chromatographie liquide haute performance, sachant que la longueur d'onde d'extraction de chacun des chromatogrammes est de 220 nm :

| t (min.) | A (%) | B (%) |
|---|---|---|
| 0 | 90 | 10 |
| 6 | 15 | 85 |
| 8 | 15 | 85 |

Eluant A : eau + 0,02% d'acide trifluoroacétique ; éluant B : acétonitrile.
Débit : 1 ml/min ; volume injecté : 5 µl ; température : 40 °C. Colonne : Uptisphère 3µm ODS, 50 x 4,6 mm i.d. (Interchim)

Les spectres de masse sont obtenus à partir d'un spectromètre de masse simple quadripôle équipé d'une source *electrospray* (Micromass, Platforme II).

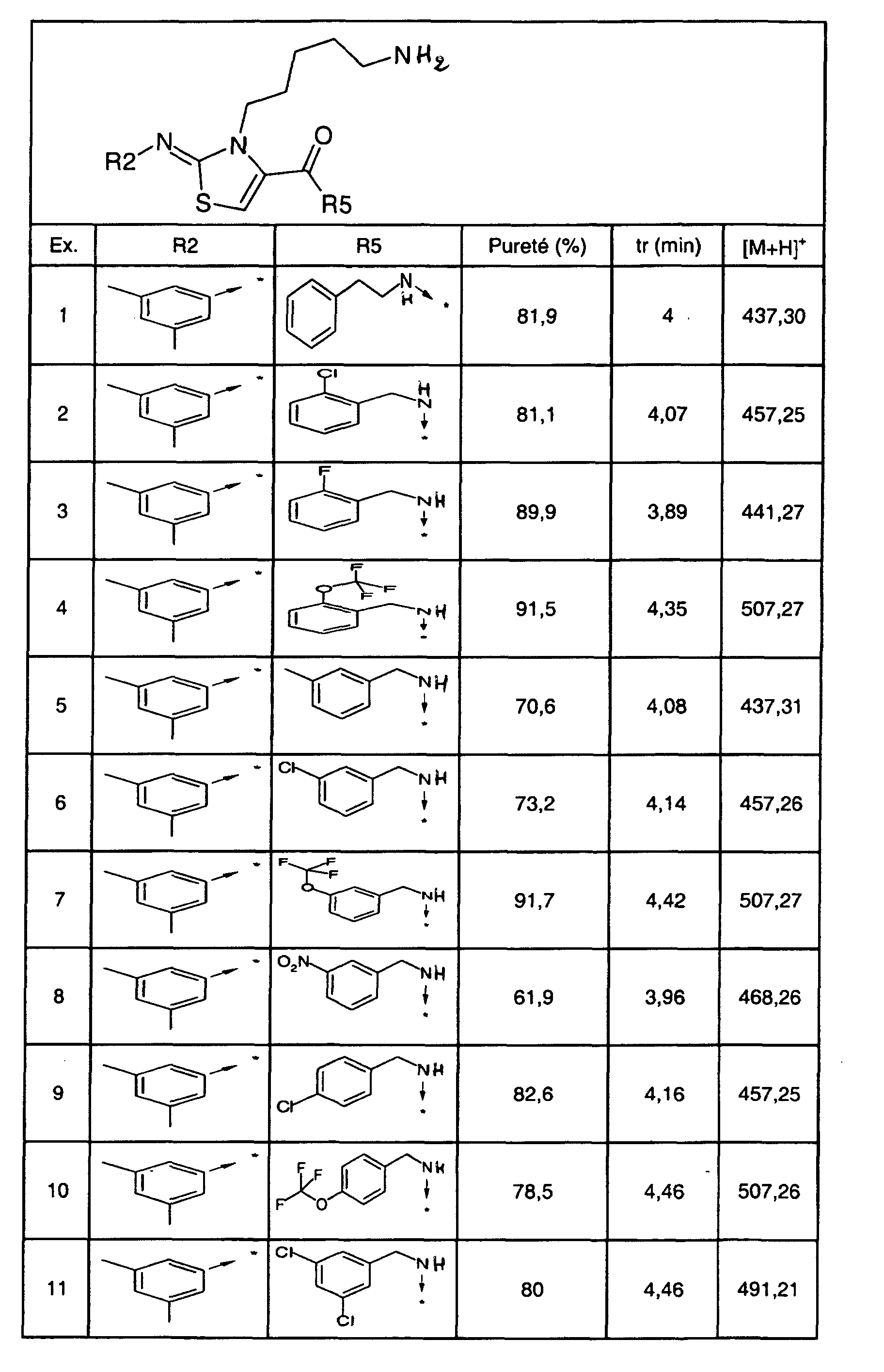

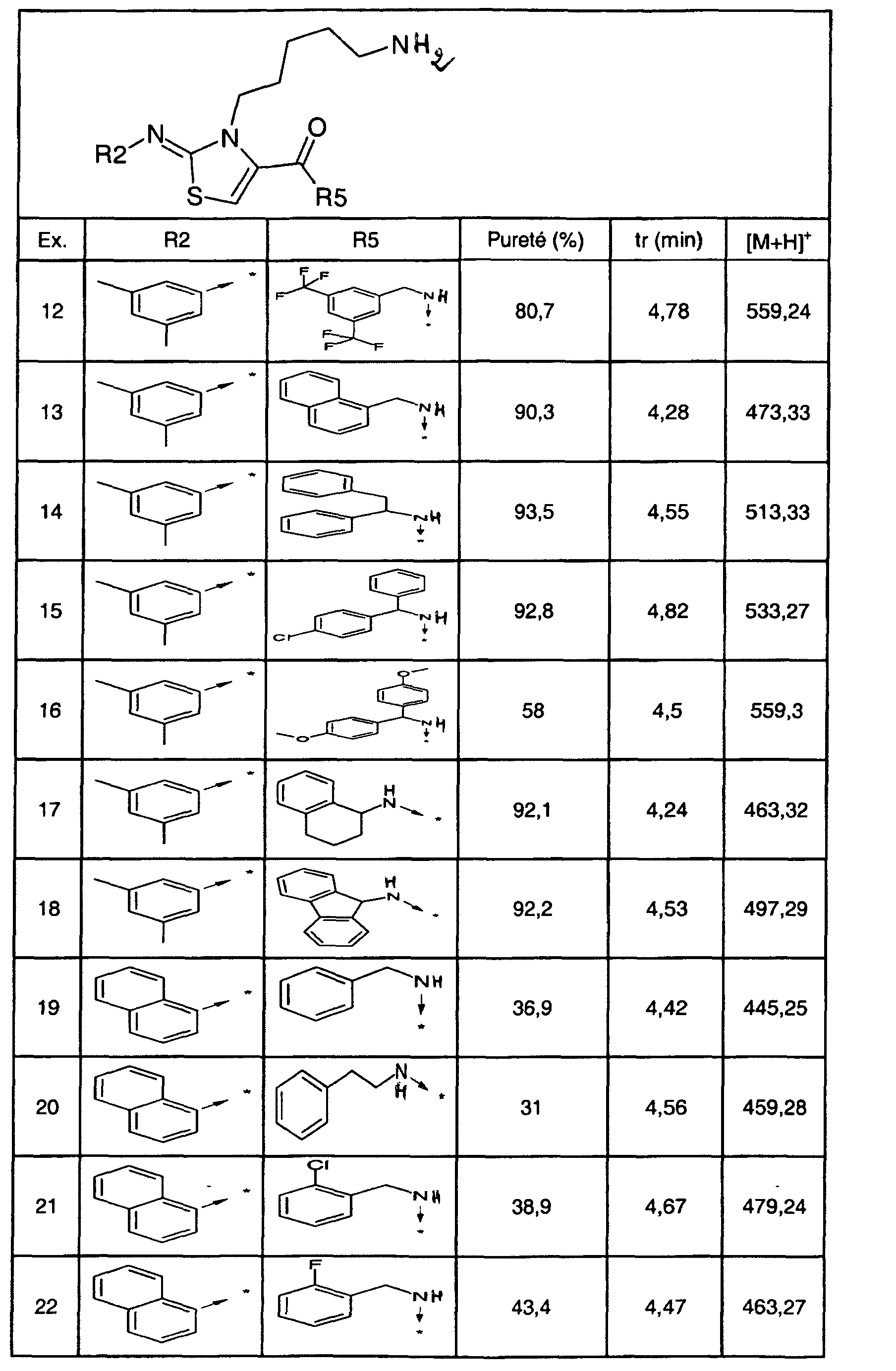

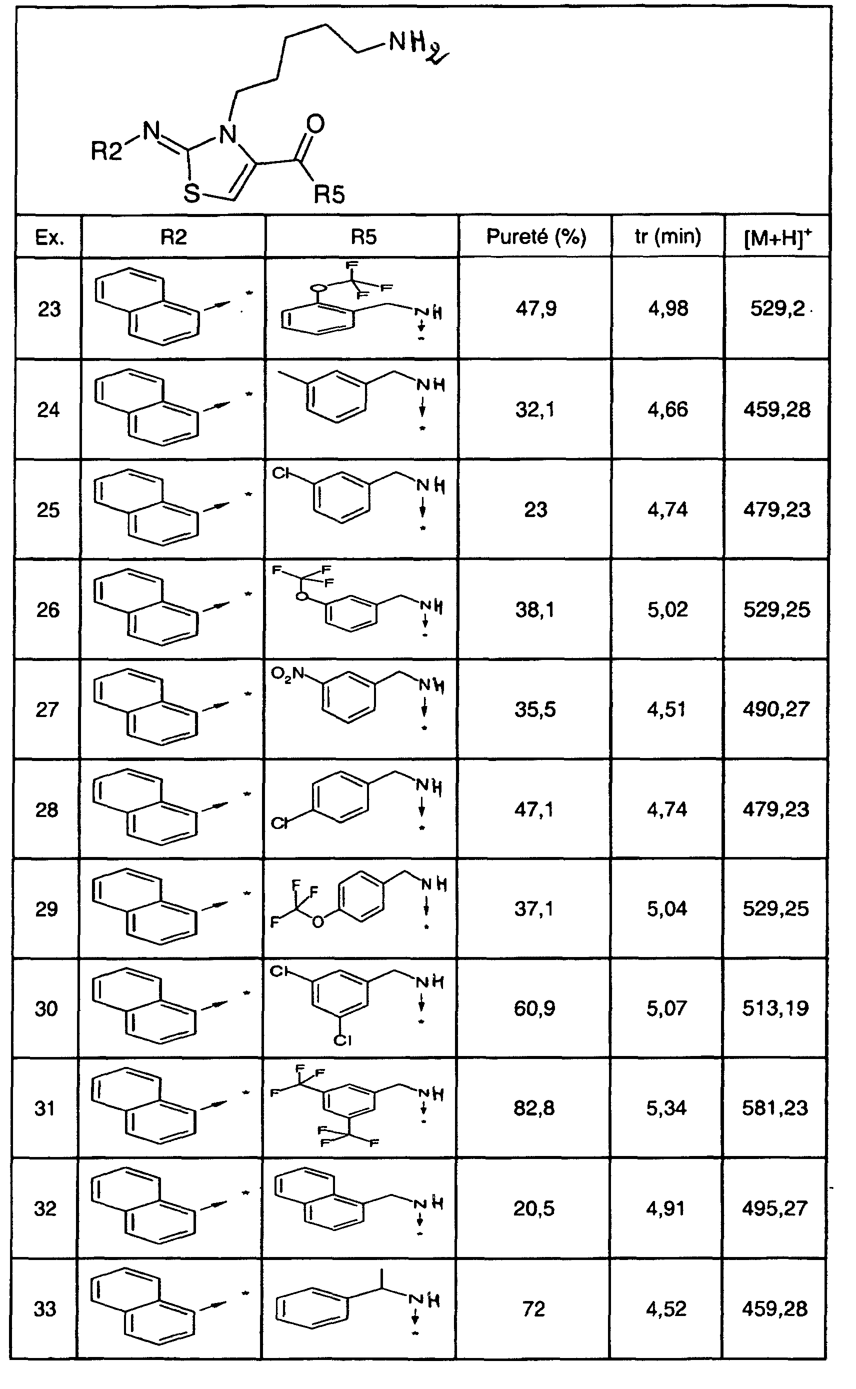

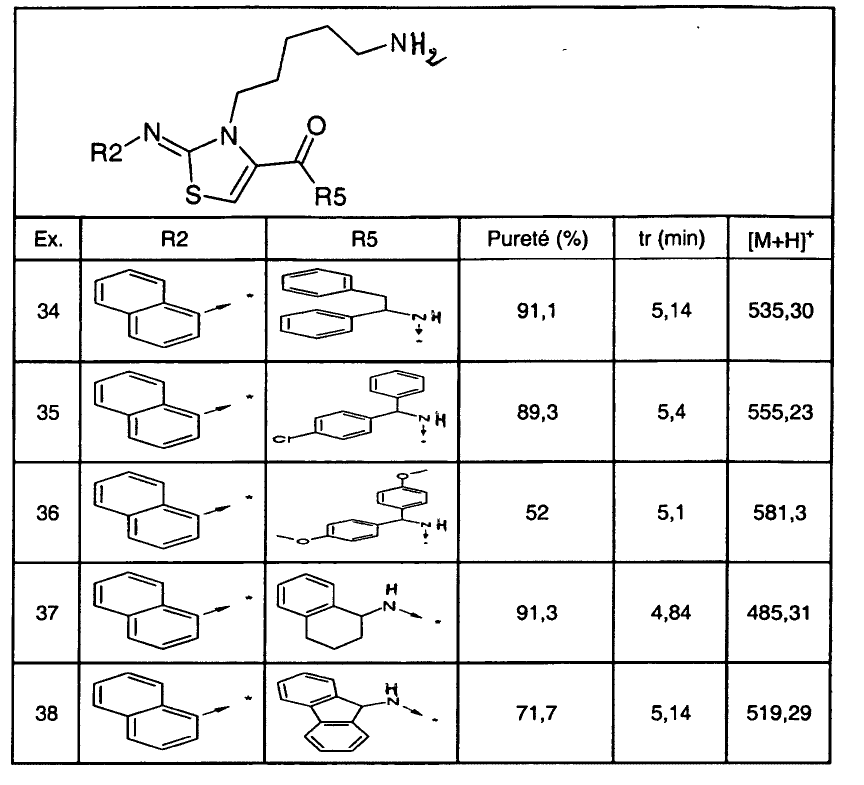

Certains composés selon l'invention peuvent être obtenus selon la méthode E décrite ci-après.

### METHODE E

### Synthèse en solution de dérivés 2-iminothiazole-4-carboxamides à partir de diamines symétriques monoprotégées (Boc)

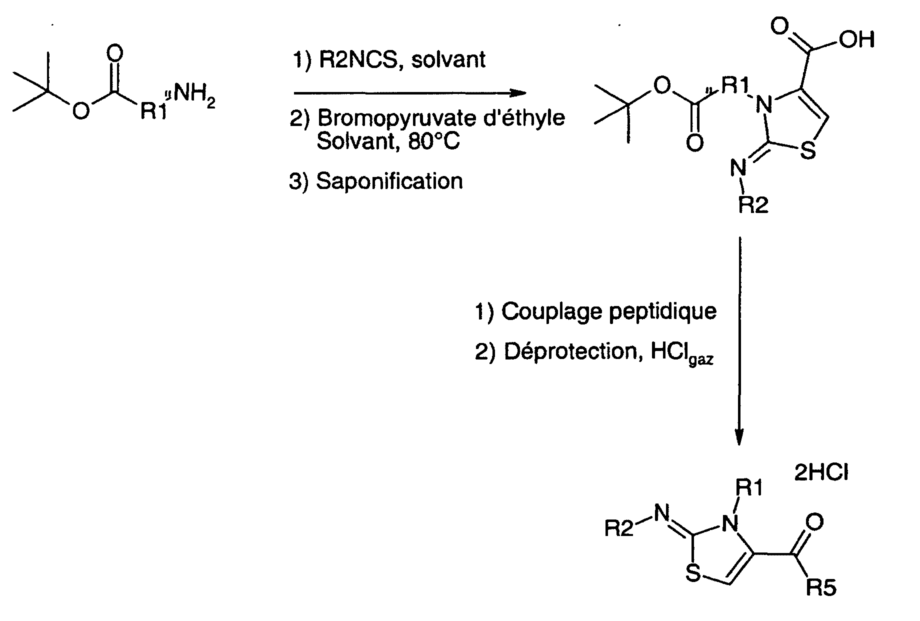

### Procédure générale :

La diamine symétrique monoprotégée (Boc) (1 equiv) est agitée durant la nuit avec un isothiocyanate aromatique (1 equiv) à température ambiante dans un solvant anhydre tel que le dioxane, le diméthylformamide ou le chloroforme. A l'intermédiaire isothiourée brut, on ajoute successivement 1 équivalent d'une base inorganique comme de l'hydrogénocarbonate de sodium ou de potassium et 1 équivalent de bromopyruvate d'éthyle préalablement dissous dans un solvant anhydre tel que le dioxane ou le diméthylformamide. Le mélange est ensuite chauffé à 80 °C pendant 1 à 3 heures et les sels inorganiques sont éliminés par filtration. Les solvants sont évaporés sous vide et le résidu est purifié par chromatographie éclair sur gel de silice en utilisant un gradient acétate d'éthyle / heptane. La saponification de l'ester intermédiaire est effectuée dans un solvant tel que le tétrahydrofuranne à l'aide d'une solution 1*N* de KOH, LiOH ou NaOH. Le mélange est agité vigoureusement pendant 6 à 20 heures à température ambiante puis acidifié avec une solution aqueuse 1*N* d'acide chlorhydrique jusqu'à pH 2,5. La phase organique est extraite plusieurs fois avec du dichlorométhane puis les phase organiques sont lavées avec de l'eau jusqu'à pH neutre et séchées sur sulfate de sodium.

A une solution de l'acide carboxylique intermédiaire (1 équiv.) et d'un agent de couplage peptidique tel que DIC, DIC/HOBt, HATU ou TBTU (1,1 à 2 équiv.), préalablement dissous dans un solvant anhydre comme le diméthylformamide, est ajoutée sous argon une amine primaire ou secondaire (1,1 à 2 équiv.) pré-dissoute dans un solvant anhydre comme le diméthylformamide. Le mélange est agité pendant la nuit à température ambiante. Le solvant est évaporé sous vide et le résidu purifié par chromatographie éclair sur gel de silice en utilisant un gradient acétate d'éthyle / heptane. Le carboxamide intermédiaire est dilué dans un solvant tel que le dichlorométhane ou l'acétate d'éthyle et déprotégé après passage dans la solution d'un courant de chlorure d'hydrogène sec pendant 1 à 6 heures à température ambiante. Le dichlorhydrate correspondant est isolé soit par filtration du précipité soit, après évaporation sous vide du solvant, par addition de diéthyléther pour une meilleure cristallisation.

### Préparation 24

### (2Z)-3-{5-[(tert-butoxycarbonyl)amino]pentyl}-2-[(3,5-diméthylphényl)imino]-2,3-dihydro-1,3-thiazole-4-carboxylate d'éthyle (C₂₄H₃₅N₃O₄S ; MM = 461,63)

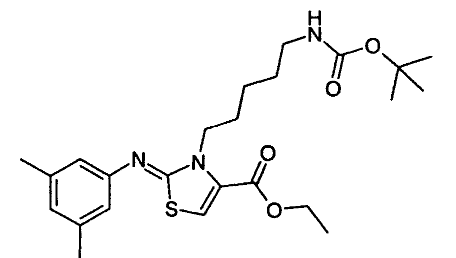

Du *N*-Boc-1,5-diaminopentane (1,04 g ; 5 mmol) est agité avec du 3,5-diméthylisothiocyanate (824 mg ; 5 mmol) dans 10 ml de dioxane anhydre. A l'intermédiaire isothiourée brut, on ajoute successivement 420 mg (5 mmol) d'hydrogénocarbonate de sodium et 1,08 g (5 mmol) de bromopyruvate d'éthyle préalablement dissous dans 2 ml de dioxane anhydre. Le mélange est ensuite chauffé à 80 °C pendant une heure et les sels inorganiques sont éliminés par filtration. Le dioxane est évaporé sous vide et le résidu jaune est purifié par chromatographie éclair sur gel de silice (éluant : acétate d'éthyle / heptane 2:8 puis 3:7). Une huile jaune (1,8 g ; rendement de 77,9%) correspondant au composé attendu est alors isolée.
RMN ¹H (DMSO-*d*_{*6*}, 400 MHz) δ: 7,23 (s, 1H) ; 6,71 (s large, 1H) ; 6,65 (s, 1H) ; 6,54 (s, 2H) ; 4,26 (q, 2H, J = 6,4 Hz) ; 4,13 (t, 2H, J = 6,4 Hz) ; 2,9 (q, 2H, J = 6 Hz) ; 2,22 (s, 6H); 1,63 (m, 2H); 1,4 (m, 2H); 1,36 (s, 9H); 1,29-1,23 (m, 2H + 3H). SM/CL : m/z = 462,3 (M+H)⁺.

### Préparation 25

### Acide (2Z)-3-{5-[(tert-butoxycarbonyl)amino]pentyl}-2-[(3,5-diméthylphényl)imino]-2,3-dihydro-1,3-thiazole-4-carboxylique (C₂₂H₃₁N₃O₄S ; MM = 433,57)

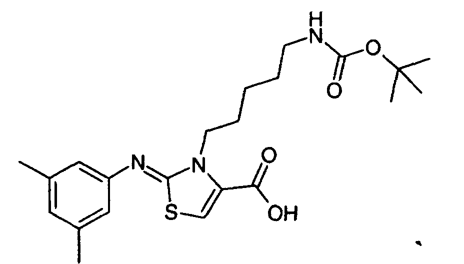

Le composé de la préparation 25 (1,77 g; 3,83 mmol) est dissous dans 20 ml de tétrahydrofuranne et traité avec 15 ml de solution aqueuse 1*N* de NaOH. Le mélange est agité vigoureusement pendant 6 heures à température ambiante. Le carboxylate est ensuite acidifié avec une solution aqueuse 1*N* d'acide chlorhydrique jusqu'à pH 2,5. La phase aqueuse est extraite avec du dichlorométhane (4 x 50 ml) et les phases organiques sont lavées avec de l'eau jusqu'à pH neutre et séchées sur sulfate de sodium. Un solide jaune pâle est isolé (1,51 g; rendement de 90,9%) après évaporation sous vide des solvants.
RMN ¹H (DMSO-*d*_{*6*}, 400 MHz) δ : 13,28 (s large, 1H) ; 7,16 (s, 1H) ; 6,69 (s large, 1H) ; 6,65 (s, 1H) ; 6,54 (s, 2H) ; 4,17 (t, 2H, J = 7,2 Hz) ; 2,89 (q, 2H, J = 6,4 Hz) ; 2,22 (s, 6H) ; 1,63 (q, 2H, J = 6,8 Hz) ; 1,41 (m, 2H) ; 1,36 (s, 9H) ; 1,25 (m, 2H). SM/CL : m/z = 434,27 (M+H)⁺.

### Préparation 26

### 5-[(2Z)-2-[(3,5-diméthylphényl)imino]-4-{[(1-phénylpropyl)amino]carbonyl}-1,3-thiazol-3(2H)-yl]pentylcarbamate de tert-butyle (C₃₁H₄₂N₄O₃S ; MM = 550,76)

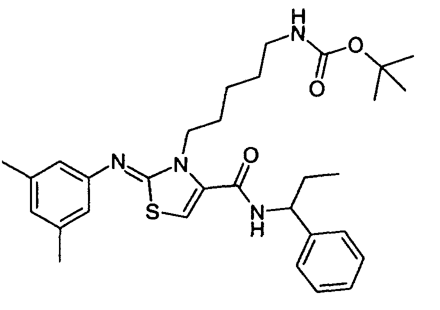

600 mg (1,38 mmol) de l'acide carboxylique de la préparation 26 sont préalablement activés avec 888 mg (2,76 mmol; 2 équiv.) de TBTU dans 10 ml de diméthylformamide anhydre pendant une heure. 410 µl (2,76 mmol ; 2 équiv.) d'α-éthylbenzylamine sont ensuite ajoutés et le mélange agité à température ambiante pendant la nuit. Après évaporation du diméthylformamide, le résidu brut est purifié par chromatographie éclair sur gel de silice (éluant : acétate d'éthyle / heptane 4:6) pour donner un solide blanc (498 mg ; rendement de 65,5%).
RMN ¹H (DMSO-*d*_{*6*}, 400 MHz) δ : 9,00 (d, 1H, J = 8,4 Hz) ; 7,36-7,30 (m, 4H) ; 7,25-7,21 (m, 1H) ; 6,72 (t, 1H, J = 5,4 Hz) ; 6,67 (s, 1H) ; 6,63 (s, 1H) ; 6,53 (s, 2H) ; 4,77 (q, 1H, J = 8.8 Hz) ; 3,95 (m, 2H) ; 2,84 (q, 2H, J = 6 Hz) ; 2,21 (s, 6H) ; 1,74 (m, 2H) ; 1,51 (m, 2H) ; 1,36 (s, 9H) ; 1,31 (q, 2H, J = 7,2 Hz) ; 1,13 (m, 2H) ; 0,89 (t, 3H, J = 7,2 Hz).
SM/CL : m/z = 551,44 (M+H)⁺.

### Exemple 39

### Dichlorhydrate de (2Z)-3-(5-aminopentyl)-2-[(3,5-diméthylphényl)imino]-N-(1-phénylpropyl)-2,3-dihydro-1,3-thiazole-4-carboxamide (C₂₆H₃₄N₄OS.2HCl ; MM = 523,57)

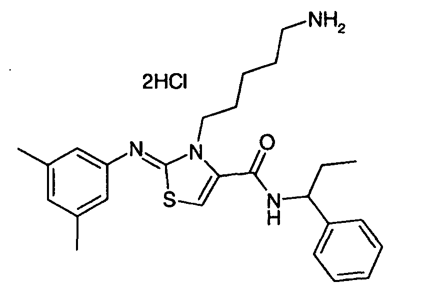

300 mg (0,54 mmol) de 5-[(2Z)-2-[(3,5-diméthylphényl)imino]-4- {[(1-phénylpropyl)amino]carbonyl}-1,3-thiazol-3(2*H*)-yl]pentylcarbamate de tert-butyle sont dissous dans 15 ml d'acétate d'éthyle. Après bullage de chlorure d'hydrogène anhydre pendant une heure à température ambiante, le sel dichlorhydrate correspondant précipite. Il est récupéré par filtration et lavé avec du diéthyléther pour donner un solide blanc (268 mg ; rendement de 94,8%).
RMN ¹H (DMSO-*d*_{*6*}, 400 MHz) δ : 9,48 (s large, 1H) ; 8,03 (s large, 3H) ; 7,39-7,32 (m, 5H) ; 7,25 (t, 1H, J = 7,2 Hz) ; 7,00 (m, 3H) ; 4,80 (q, 1H, J = 8,4 Hz) ; 4,33 (s large, 2H) ; 2,70 (q, 2H, J = 6,8 Hz) ; 2,29 (s, 6H) ; 1,77 (m, 2H) ; 1,65 (m, 2H) ; 1,52 (m, 2H) ; 1,27 (m, 2H) ; 0,89 (t, 3H, J = 7,2 Hz).
SM/CL : m/z = 451,35 (M+H)⁺.

Les composés repris dans le tableau ci-après ont été synthétisés en utilisant la méthode E.

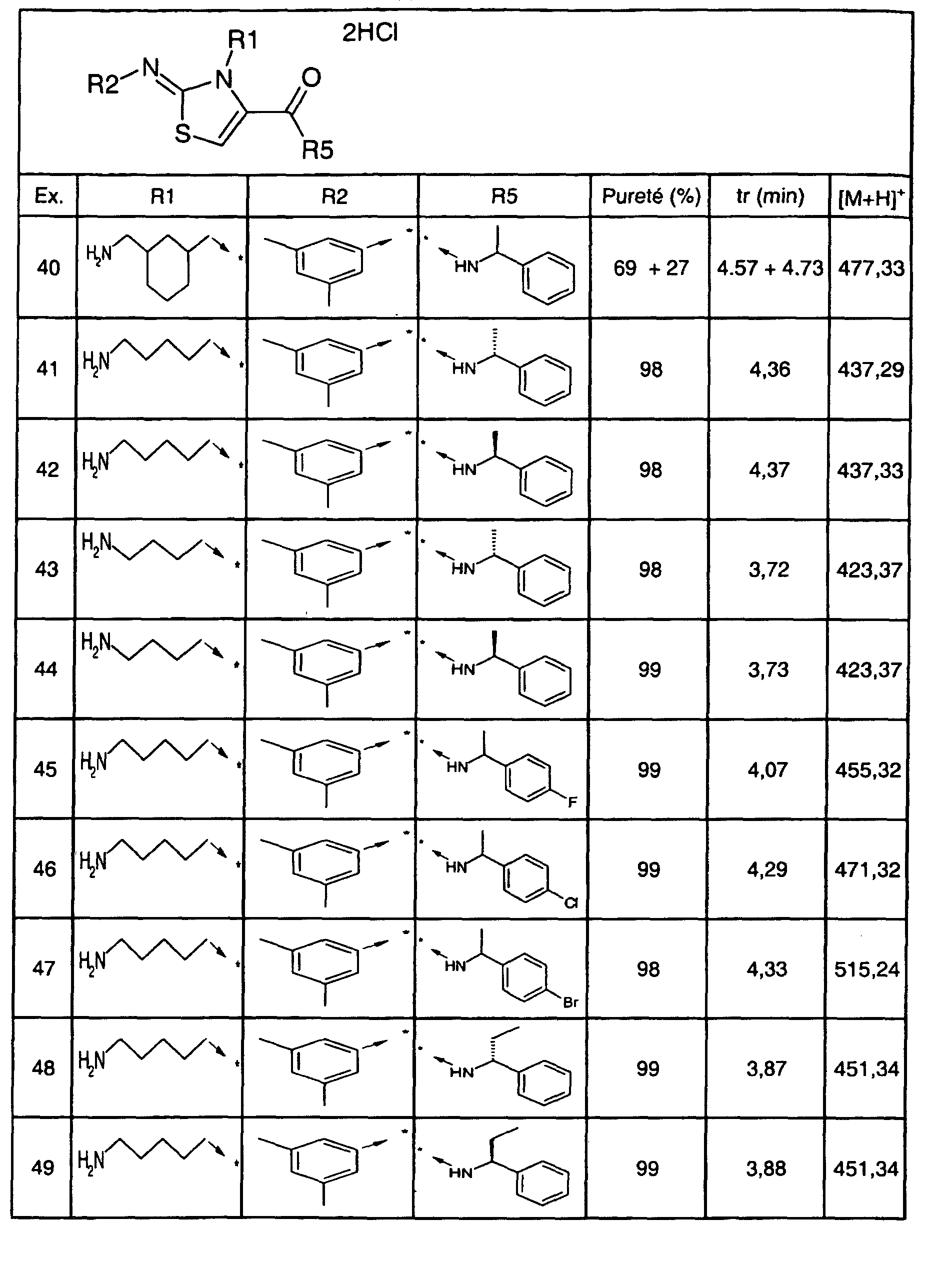

### PROPRIETES PHARMACOLOGIQUES DES PRODUITS DE L'INVENTION

Les composés de la présente invention peuvent et ont été testés en ce qui concerne leur affinité pour différents sous-types de récepteurs de la somatostatine selon les procédures décrites ci-après.

### Etude de l'affinité pour les sous-types de récepteurs de la somatostatine humaine :

L'affinité d'un composé de l'invention pour les sous-types de récepteurs de la somatostatine 1 à 5 (sst₁, sst₂, sst₃, sst₄ et sst₅, respectivement) est déterminée par la mesure de l'inhibition de la liaison de [¹²⁵I-Tyr¹¹]SRIF-14 à des cellules transfectées CHO-K1.

Le gène du récepteur sst₁ de la somatostatine humaine a été cloné sous forme d'un fragment génomique. Un segment *Pst*I-*Xmn*I de 1,5 Kb contenant 100 pb de la région 5' non transcrite, 1,17 Kb de la région codante en totalité, et 230 bp de la région 3' non transcrite est modifié par l'addition du linker Bg1II. Le fragment d'ADN résultant est souscloné dans le site *BamH*I d'un pCMV-81 pour donner le plasmide d'expression chez les mammifères (fourni par Dr. Graeme Bell, Univ. Chicago). Une lignée de cellules clonées exprimant de façon stable le récepteur sst₁ est obtenue par transfection dans des cellules CHO-K1 (ATCC) grâce à la méthode de co-précipitation calcium phosphate. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Le gène du récepteur sst₂ de la somatostatine humaine, isolé sous forme d'un fragment génomique d'ADN de 1.7 Kb *Bam*HI-*Hind*III et souscloné dans un vecteur plasmidique pGEM3Z (Promega), a été fourni par le Dr. G. Bell (Univ. of Chicago). Le vecteur d'expression des cellules de mammifères est construit en insérant le fragment *Bam*H1-*Hind*II de 1,7 Kb dans des sites de restriction endonucléase compatibles du plasmide pCMV5. Une lignée de cellules clonées est obtenue par transfection dans des cellules CHO-K1 grâce à la méthode de co-précipitation calcium phosphate. Le plasmide pRSV-neo est inclus comme marqueur de sélection.

Le récepteur sst₃ est isolé comme fragment génomique, et la séquence codante complète est contenue dans un fragment *Bam*HI/*Hind*III de 2,4 Kb. Le plasmide d'expression chez les mammifères, pCMV-h3, est construit par insertion du fragment *Nco*I-*Hind*III de 2,0 Kb dans le site EcoR1 du vecteur pCMV après modification des terminaisons et addition de linkers EcoR1. Une lignée de cellules clonées exprimant de façon stable le récepteur sst₃ est obtenue par transfection dans des cellules CHO-K1 (ATCC) par la méthode de co-précipitation au phosphate de calcium. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Le plasmide d'expression du récepteur sst₄ humain, pCMV-HX, a été fourni par le Dr. Graeme Bell (Univ. Chicago). Ce vecteur contient le fragment génomique codant pour le récepteur sst₄ humain de 1,4 Kb *Nhe*I*-Nhe*I, 456 pb de la région 5' non transcrite, et 200 pb de la région 3' non transcrite, cloné dans les sites *Xba*I/*Eco*R1 de PCMV-HX. Une lignée de cellules clonées exprimant de façon stable le récepteur sst₄ est obtenue par transfection dans des cellules CHO-K1 (ATCC) par la méthode de co-précipitation au phosphate de calcium. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Le gène correpondant au récepteur sst₅ humain, obtenu par la méthode PCR en utilisant un clone génomique λ comme sonde, a été fourni par le Dr. Graeme Bell (Univ. Chicago). Le fragment PCR résultant de 1,2 Kb contient 21 paires de bases de la région 5' non transcrites, la région codante en totalité, et 55 pb de la région 3' non transcrite. Le clone est inséré dans un site EcoR1 du plasmide pBSSK(+). L'insert est récupéré sous la forme d'un fragment *Hind*III*-Xba*I de 1,2 Kb pour sousclonage dans un vecteur d'expression chez les mammifères, pCVM5. Une lignée de cellules clonées exprimant de façon stable le récepteur sst₅ est obtenue par transfection dans des cellules CHO-K1 (ATCC) par la méthode de co-précipitation au phosphate de calcium. Le plasmide pRSV-neo (ATCC) est inclus comme marqueur de sélection. Des lignées de cellules clonées ont été sélectionnées dans un milieu RPMI 1640 contenant 0,5 mg/ml de G418 (Gibco), clonées en cercle, et multipliées en culture.

Les cellules CHO-K1 exprimant de façon stable l'un des récepteurs sst humain sont cultivées dans un milieu RPMI 1640 contenant 10% de sérum foetal de veau et 0,4 mg/ml de généticine. Les cellules sont collectées avec de l'EDTA 0,5 mM et centrifugées à 500 g pendant environ 5 min à environ 4 °C. Le centrifugat est re-suspendu dans un milieu tampon 50 mM Tris à pH 7,4 et centrifugé deux fois à 500 g pendant environ 5 min à environ 4 °C. Les cellules sont lysées par sonication et centrifugées à 39000 g pendant environ 10 min à 4 °C. Le centrifugat est re-suspendu dans le même milieu tampon et centrifugé at 50000 g pendant 10 min à environ 4 °C et les membranes dans le centrifugat obtenu sont stockées à - 80 °C.

Des tests d'inhibition compétitive de liaison avec [¹²⁵I-Tyr¹¹]SRIF-14 sont effectués en double à l'aide de plaques en polypropylène de 96 puits. Les membranes cellulaires (10 µg protéine/puits) sont incubées avec [¹²⁵I-Tyr¹¹]SRIF-14 (0,05 nM) pendant environ 60 min à environ 37 °C dans un milieu tampon 50 mM HEPES (pH 7,4) comprenant 0,2% BSA, 5 mM de MgCl₂, 200 KIU/ml de Trasylol, 0,02 mg/ml de bacitracine et 0,02 mg/ml de fluorure de phénylméthylsulphonyle.

La [¹²⁵I-Tyr¹¹]SRIF-14 liée est séparée de la [¹²⁵I-Tyr¹¹]SRIF-14 libre par filtration immédiate à travers des plaques filtres en fibre de verre GF/C (Unifilter, Packard) préimprégné avec 0,1 % de polyéthylènimine (P.E.I.), en utilisant un Filtermate 196 (Packard). Les filtres sont lavés avec du tampon 50 mM HEPES à environ 0-4 °C pendant environ 4 secondes et leur radioactivité est déterminée à l'aide d'un compteur (Packard Top Count).

La liaison spécifique est obtenue en soustrayant la liaison non spécifique (déterminée en présence de 0,1 µM SRIF-14) de la liaison totale. Les données relatives à la liaison sont analysées par analyse en régression non-linéaire assistée par ordinateur (MDL) et les valeurs des constantes d'inhibition (Ki) values sont déterminées.

La détermination du caractère agoniste ou antagoniste d'un composé de la présente invention est effectuée à l'aide du test décrit ci-après.

### Test fonctionnel : Inhibition de la production d'AMPc intracellulaire :

Des cellules CHO-K1 exprimant les sous-types de récepteurs de la somatostatine humaine (SRIF-14) sont cultivées dans des plaques à 24 puits dans un milieu RPMI 1640 avec 10% de sérum foetal de veau et 0,4 mg/ml de généticine. Le milieu est changé le jour précédant l'expérience.

Les cellules à raison de 10⁵ cellules/puits sont lavées 2 fois avec 0,5 ml de nouveau milieu RPMI comprenant 0,2 % BSA complété par 0,5 mM de 3-isobutyl-1-méthylxanthine (IBMX) et incubées pendant environ 5 min à environ 37 °C.
□ La production d'AMP cyclique est stimulée par l'addition de 1 mM de forskoline (FSK) pendant 15-30 minutes à environ 37 °C.
□ L'effet inhibiteur de la somatostatine d'un composé agoniste est mesuré par l'addition simultanée de FSK (1µM) , SRIF-14 (10⁻¹² M to 10⁻⁶ M) et du composé à tester (10⁻¹⁰ M à 10⁻⁵ M).
□ L'effet antagoniste d'un composé est mesuré par l'addition simultanée de FSK (1µM), SRIF-14 (1 to 10 nM) et du composé à tester (10⁻¹⁰ M to 10⁻⁵ M).

Le milieu réactionnel est éliminé et 200 ml de HCl 0,1 N sont ajoutés. La quantité d'AMPc est mesurée par un test radioimmunologique (Kit FlashPlate SMP001A, New England Nuclear).

### Résultats :

Les tests effectués selon les protocoles décrits ci-dessus ont permis de montrer que chacun des composés des exemples 40 à 49 présente une constante Kᵢ inférieure à 200 nM.

## Revendications

1. Composé **caractérisé en ce qu'**il correspond :
• à la formule 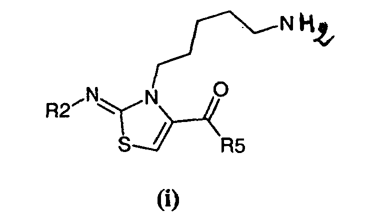 dans laquelle :
- R2 représente le radical 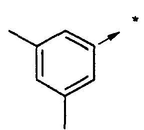 et R5 représente le radical 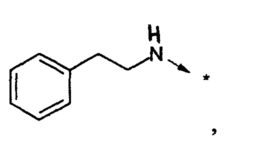
- R2 représente le radical 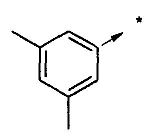 et R5 représente le radical 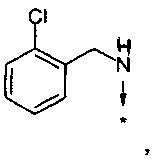
- R2 représente le radical 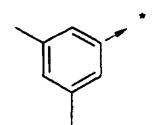 et R5 représente le radical 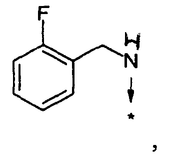
- R2 représente le radical 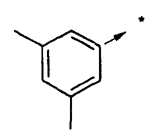 et R5 représente le radical 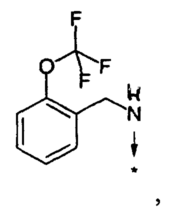
- R2 représente le radical 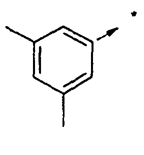 et R5 représente le radical 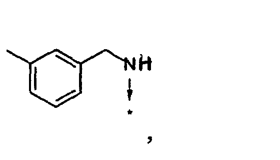
- R2 représente le radical 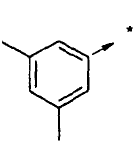 et R5 représente le radical 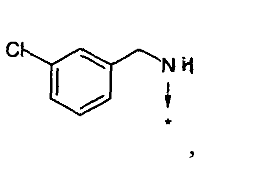
- R2 représente le radical 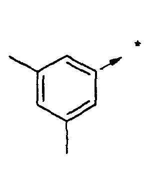 et R5 représente le radical 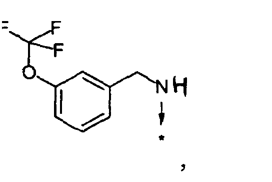
- R2 représente le radical 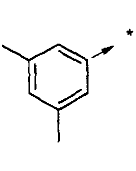 et R5 représente le radical 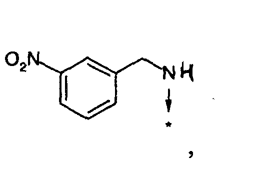
- R2 représente le radical 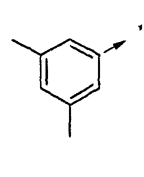 et R5 représente le radical 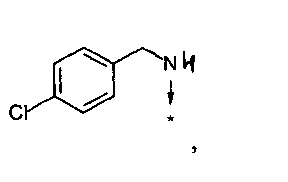
- R2 représente le radical 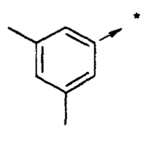 et R5 représente le radical 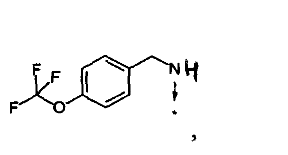
- R2 représente le radical 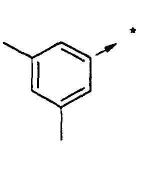 et R5 représente le radical 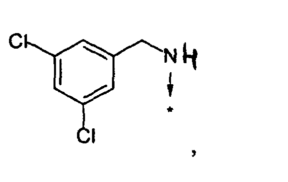
- R2 représente le radical 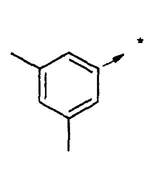 et R5 représente le radical 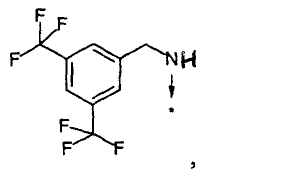
- R2 représente le radical 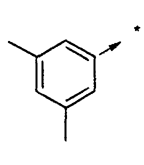 et R5 représente le radical 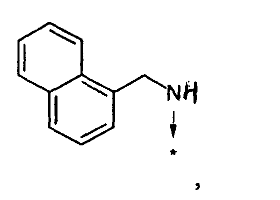
- R2 représente le radical 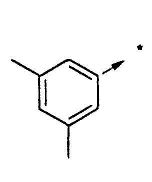 et R5 représente le radical 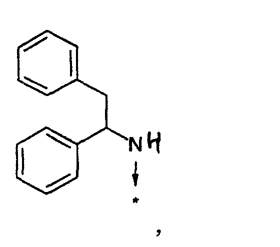
- R2 représente le radical 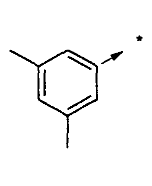 et R5 représente le radical 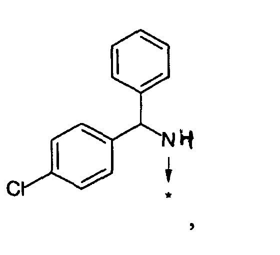
- R2 représente le radical 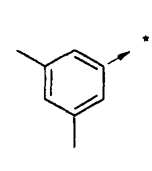 et R5 représente le radical 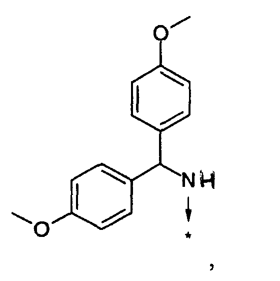
- R2 représente le radical 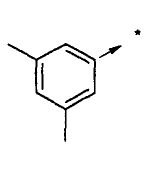 et R5 représente le radical 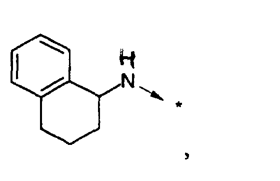
- R2 représente le radical 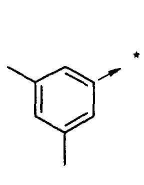 et R5 représente le radical 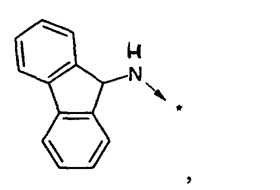
- R2 représente le radical 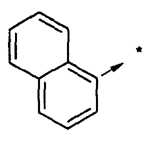 et R5 représente le radical 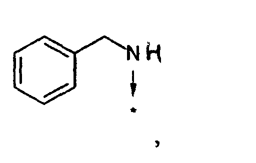
- R2 représente le radical 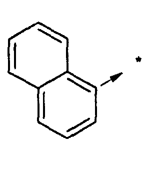 et R5 représente le radical 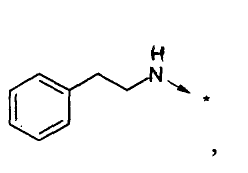
- R2 représente le radical 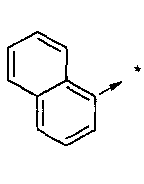 et R5 représente le radical 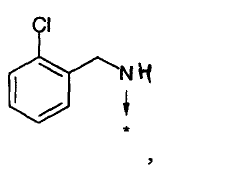
- R2 représente le radical 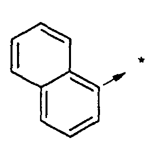 et R5 représente le radical 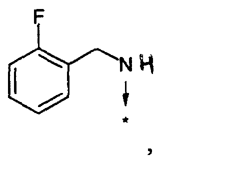
- R2 représente le radical 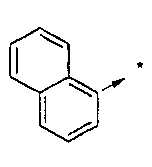 et R5 représente le radical 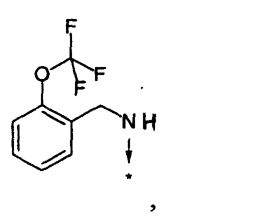
- R2 représente le radical 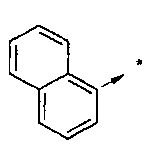 et R5 représente le radical 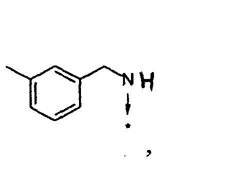
- R2 représente le radical 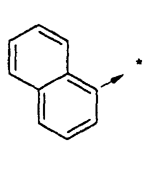 et R5 représente le radical 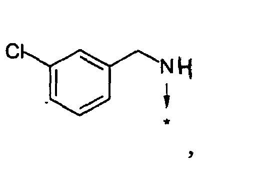
- R2 représente le radical 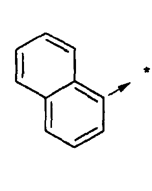 et R5 représente le radical 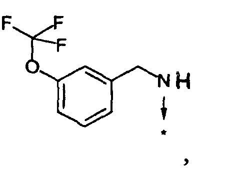
- R2 représente le radical 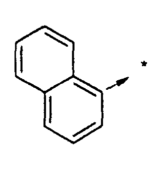 et R5 représente le radical 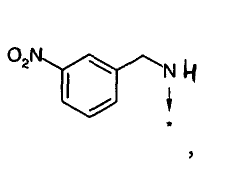
- R2 représente le radical 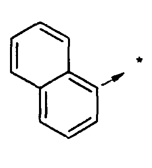 et R5 représente le radical 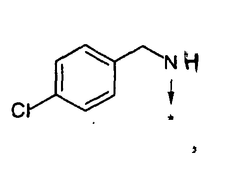
- R2 représente le radical 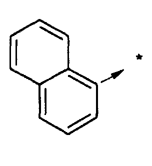 et R5 représente le radical 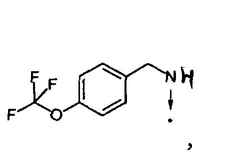
- R2 représente le radical 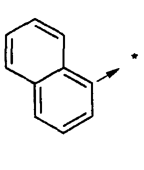 et R5 représente le radical 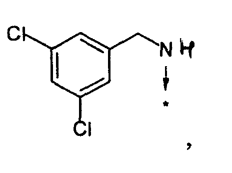
- R2 représente le radical 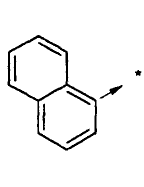 et R5 représente le radical 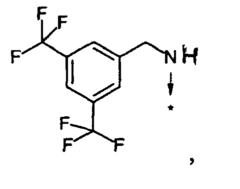
- R2 représente le radical 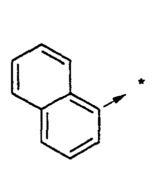 et R5 représente le radical 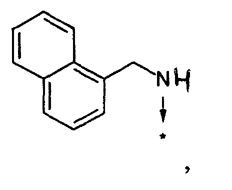
- R2 représente le radical 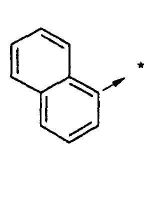 et R5 représente le radical 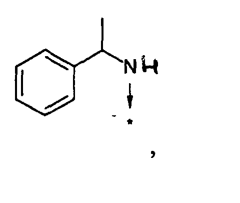
- R2 représente le radical 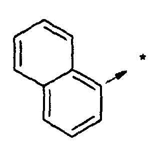 et R5 représente le radical 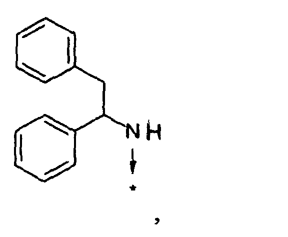
- R2 représente le radical 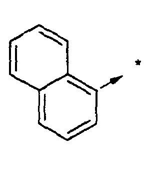 et R5 représente le radical 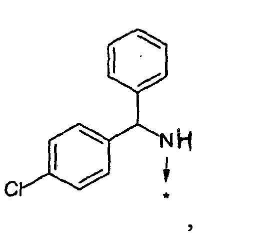
- R2 représente le radical 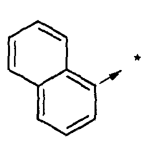 et R5 représente le radical 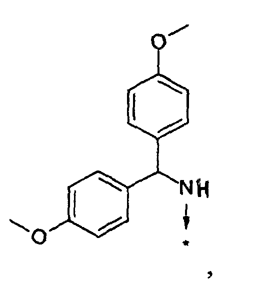
- R2 représente le radical 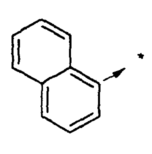 et R5 représente le radical 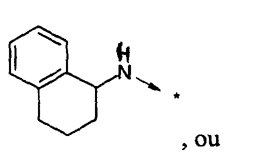 enfin
- R2 représente le radical 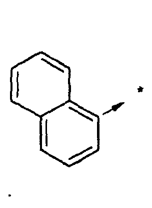 et R5 représente le radical 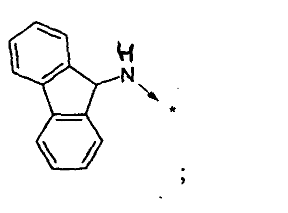
• ou à la formule 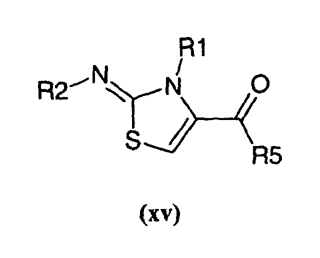 dans laquelle:
- R1 représente 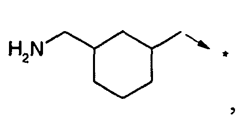 R2 représente 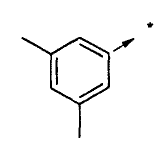 et R5 représente 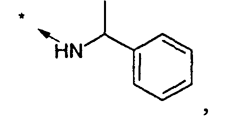
- R1 représente 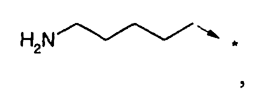 R2 représente 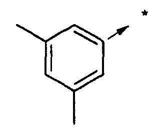 et R5 représente 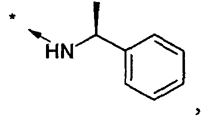
- R1 représente 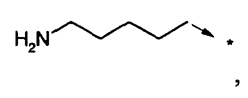 R2 représente 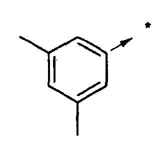 et R5 représente 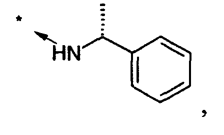
- R1 représente 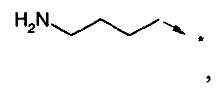 R2 représente 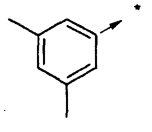 et R5 représente 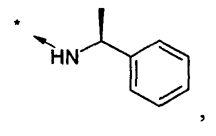
- R1 représente 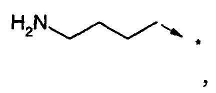 R2 représente 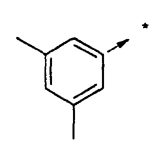 et R5 représente 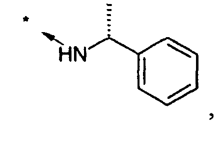
- R1 représente 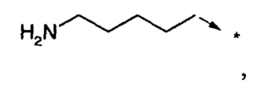 R2 représente 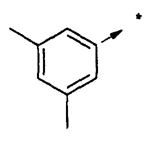 et R5 représente 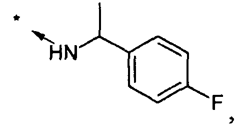
- R1 représente 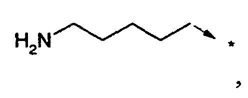 R2 représente 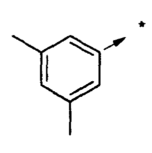 et R5 représente 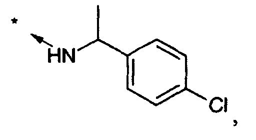
- R1 représente 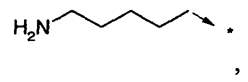 R2 représente 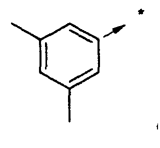 et R5 représente 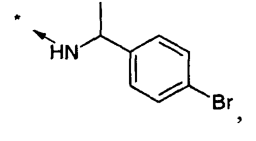
- R1 représente 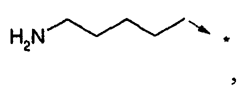 R2 représente 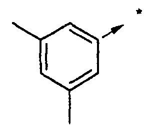 et R5 représente 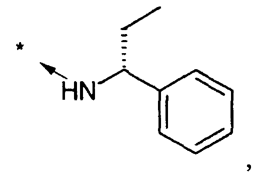
ou enfin
- R1 représente 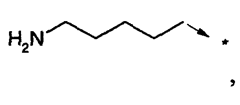 R2 représente 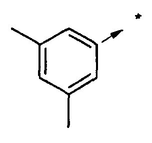 et R5 représente 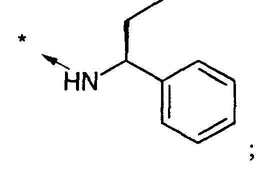
ou sel d'un de ces composés.

2. Composé selon la revendication 1, **caractérisé en ce qu'**il répond à la formule 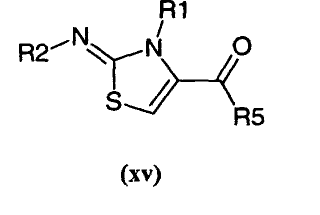 dans laquelle:
- R1 représente 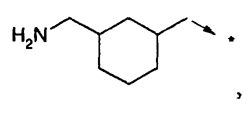 R2 représente 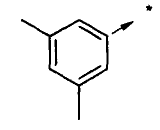 et R5 représente 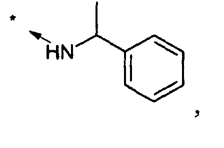
- R1 représente 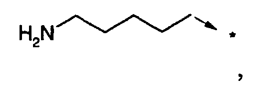 R2 représente 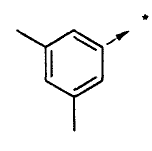 et R5 représente 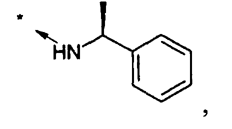
- R1 représente 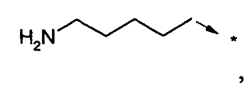 R2 représente 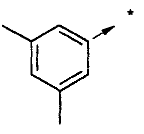 et R5 représente 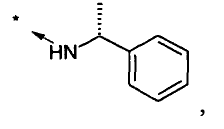
- R1 représente 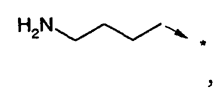 R2 représente 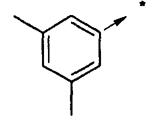 et R5 représente 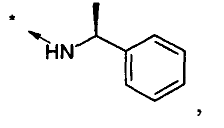
- R1 représente 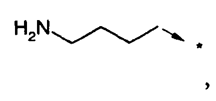 R2 représente 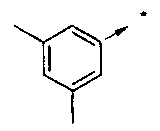 et R5 représente 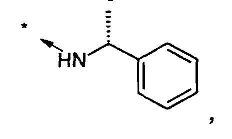
- R1 représente 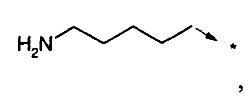 R2 représente 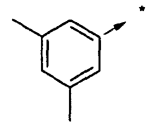 et R5 représente 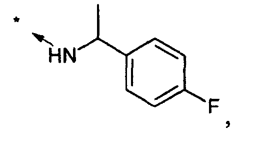
- R1 représente 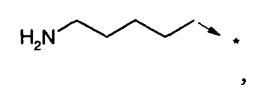 R2 représente 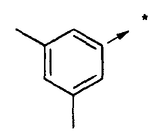 et R5 représente 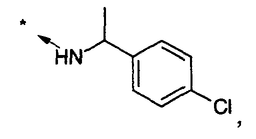
- R1 représente 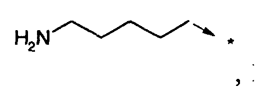 R2 représente 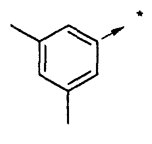 et R5 représente 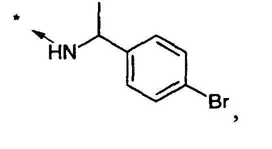
- R1 représente 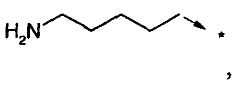 R2 représente 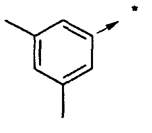 et R5 représente 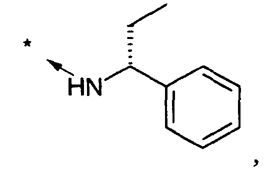
ou enfin
- R1 représente 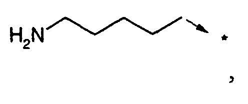 R2 représente 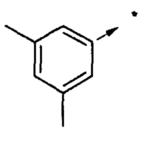 et R5 représente 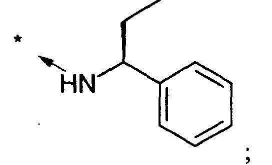
ou sel d'un de ces composés.

3. A titre de médicament, un composé selon la revendication 1 ou 2 ou un sel pharmaceutiquement acceptable dudit composé.

4. Composition pharmaceutique comprenant, à titre de principe actif, un composé selon la revendication 1 ou 2 ou un sel pharmaceutiquement acceptable dudit composé.

5. Utilisation d'un composé selon la revendication 1 ou 2 ou d'un sel pharmaceutiquement acceptable dudit composé pour préparer un médicament destiné à traiter les états pathologiques ou les maladies dans lesquels un (ou plusieurs) des récepteurs de la somatostatine est (sont) impliqué(s).

6. Utilisation selon la revendication 5, **caractérisée en ce que** les états pathologiques ou les maladies à traiter sont choisis parmi le groupe composé des états pathologiques ou des maladies qui suivent : l'acromégalie, les adénomes hypophysaires, la maladie de Cushing, les gonadotrophinomes et les prolactinomes, les effets secondaires cataboliques des glucocorticoïdes, le diabète insulinodépendant, la rétinopathie diabétique, la néphropathie diabétique, le syndrome X, le phénomène de Dawn, l'angiopathie, l'angioplastie, l'hyperthyroïdie, le gigantisme, les tumeurs gastroentéropancréatiques endocriniennes dont le syndrome carcinoïde, le VIPome, l'insulinome, la nésidioblastose, l'hyperinsulinémie, le glucagonome, le gastrinome et le syndrome de Zollinger-Ellison, le GRFome ainsi que le saignement aigu des varices oesophagiennes, les ulcères, le reflux gastrooesophagien, le reflux gastroduodénal, la pancréatite, les fistules entérocutanées et pancréatiques mais aussi les diarrhées, les diarrhées réfractaires du syndrome d'immunodépression acquise, la diarrhée chronique sécrétoire, la diarrhée associée avec le syndrome de l'intestin irrité, les diarrhées induites par la chimiothérapie, les troubles liés au peptide libérateur de gastrine, les pathologies secondaires aux greffes intestinales, l'hypertension portale ainsi que les hémorragies des varices chez des malades avec cirrhose, l'hémorragie gastro-intestinale, l'hémorragie de l'ulcère gastroduodénale, le saignement des vaisseaux greffés, la maladie de Crohn, les scléroses systémiques, le dumping syndrome, le syndrome du petit intestin, l'hypotension, la sclérodermie et le carcinome thyroïdien médullaire, les maladies liées à l'hyperprolifération cellulaire comme les cancers et plus particulièrement le cancer du sein, le cancer de la prostate, le cancer thyroïdien ainsi que le cancer pancréatique et le cancer colorectal, les fibroses et plus particulièrement la fibrose du rein, la fibrose du foie, la fibrose du poumon, la fibrose de la peau, également la fibrose du système nerveux central ainsi que celle du nez et la fibrose induite par la chimiothérapie, et, dans d'autres domaines thérapeutiques, les céphalées y compris les céphalées associées aux tumeurs hypophysaires, les douleurs, les désordres inflammatoires comme l'arthrite, les accès de panique, la chimiothérapie, la cicatrisation des plaies, l'insuffisance rénale résultant d'un retard de croissance, l'hyperlipidémie, l'obésité et le retard de croissance lié à l'obésité, le retard de croissance utérin, la dysplasie du squelette, le syndrome de Noonan, le syndrome d'apnée du sommeil, la maladie de Graves, la maladie polykystique des ovaires, les pseudokystes pancréatiques et ascites, la leucémie, le méningiome, la cachexie cancéreuse, l'inhibition des H pylori, le psoriasis, le rejet chronique des allogreffes ainsi que la maladie d'Alzheimer et enfin l'ostéoporose.

7. Utilisation selon la revendication 6, **caractérisée en ce que** les états pathologiques ou les maladies à traiter sont choisis parmi le groupe composé des états pathologiques ou des maladies qui suivent : l'acromégalie, les adénomes hypophysaires ou les tumeurs gastroentéropancréatiques endocriniennes dont le syndrome carcinoïde, et les saignements gastro-intestinaux.

8. Utilisation selon la revendication 6, **caractérisée en ce que** l'état pathologique ou la maladie à traiter est l'acromégalie.

9. Utilisation selon la revendication 6, **caractérisée en ce que** les états pathologiques ou les maladies à traiter sont choisis parmi les adénomes hypophysaires et les tumeurs gastroentéropancréatiques endocriniennes.

10. Utilisation selon la revendication 6, **caractérisée en ce que** les états pathologiques ou les maladies à traiter sont choisis parmi les saignements gastro-intestinaux.

## Patentansprüche

1. Verbindung, **dadurch gekennzeichnet, dass** sie
• der Formel 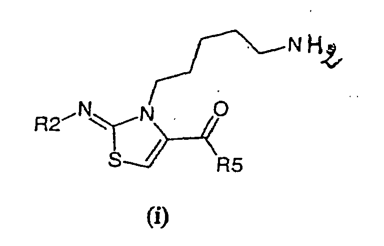 entspricht, in der
- R2 den Rest 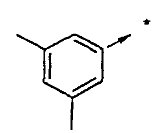 und R5 den Rest 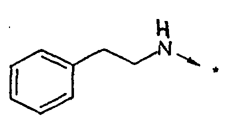 darstellt,
- R2 den Rest 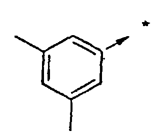 und R5 den Rest 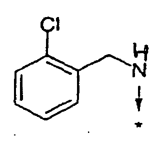 darstellt,
- R2 den Rest 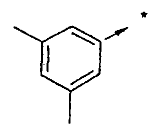 und R5 den Rest 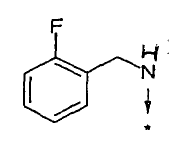 darstellt,
- R2 den Rest 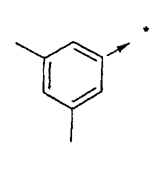 und R5 den Rest 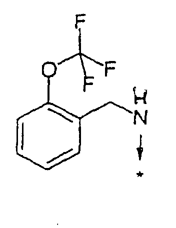 darstellt,
- R2 den Rest 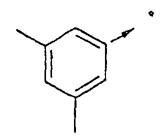 und R5 den Rest 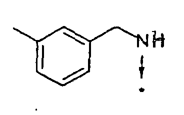 darstellt,
- R2 den Rest 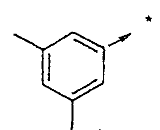 und R5 den Rest 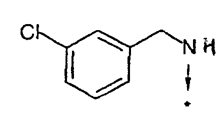 darstellt,
- R2 den Rest 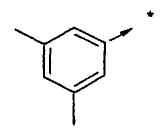 und R5 den Rest 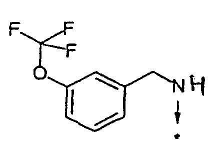 darstellt,
- R2 den Rest 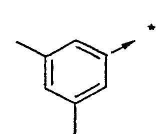 und R5 den Rest 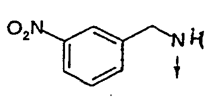 darstellt,
- R2 den Rest 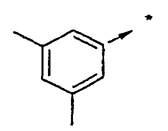 und R5 den Rest 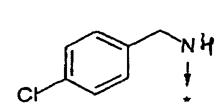 darstellt,
- R2 den Rest 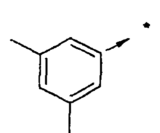 und R5 den Rest 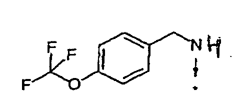 darstellt,
- R2 den Rest 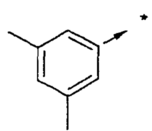 und R5 den Rest 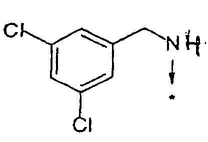 darstellt,
- R2 den Rest 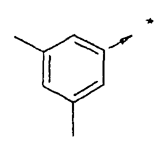 und R5 den Rest 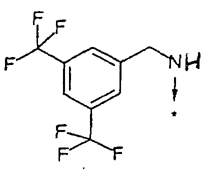 darstellt,
- R2 den Rest 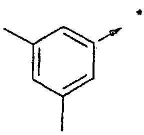 und R5 den Rest 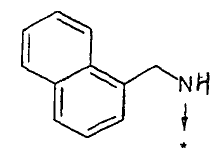 darstellt,
- R2 den Rest 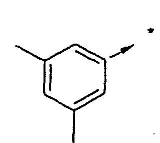 und R5 den Rest 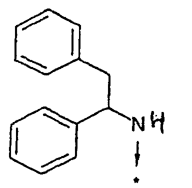 darstellt,
- R2 den Rest 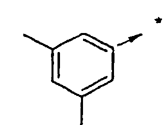 und R5 den Rest 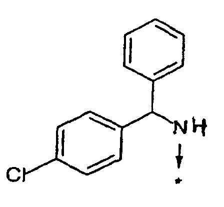 darstellt,
- R2 den Rest 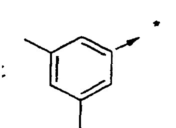 und R5 den Rest 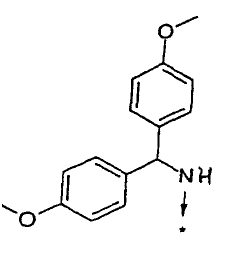 darstellt,
- R2 den Rest 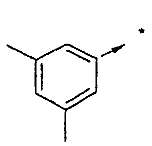 und R5 den Rest 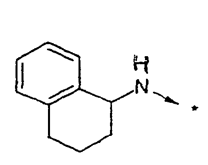 darstellt,
- R2 den Rest 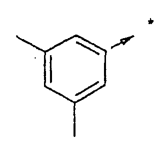 und R5 den Rest 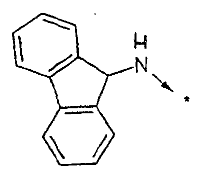 darstellt,
- R2 den Rest 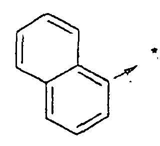 und R5 den Rest 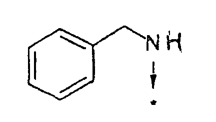 darstellt,
- R2 den Rest 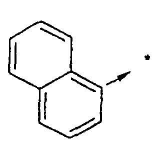 und R5 den Rest 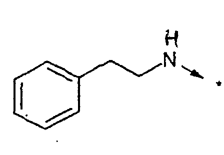 darstellt,
- R2 den Rest 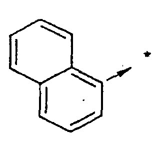 und R5 den Rest 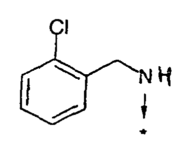 darstellt,
- R2 den Rest 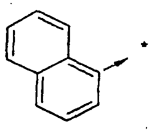 und R5 den Rest 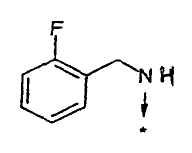 darstellt,
- R2 den Rest 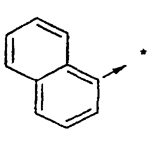 und R5 den Rest 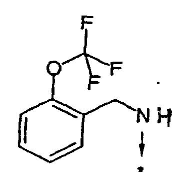 darstellt,
- R2 den Rest 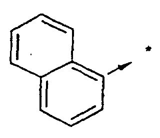 und R5 den Rest 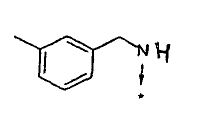 darstellt,
- R2 den Rest 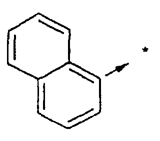 und R5 den Rest 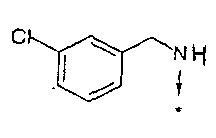 darstellt,
- R2 den Rest 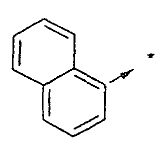 und R5 den Rest 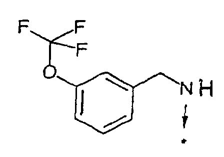 darstellt,
- R2 den Rest 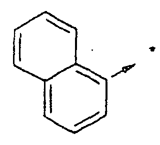 und R5 den Rest 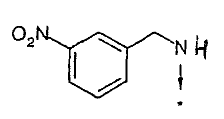 darstellt,
- R2 den Rest 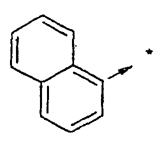 und R5 den Rest 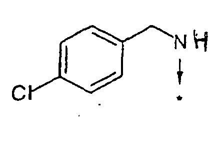 darstellt,
- R2 den Rest 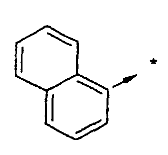 und R5 den Rest 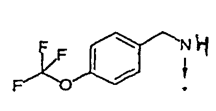 darstellt,
- R2 den Rest 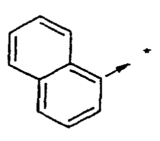 und R5 den Rest 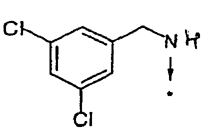 darstellt,
- R2 den Rest 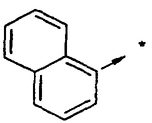 und R5 den Rest 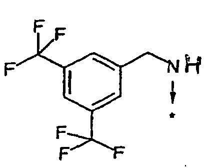 darstellt,
- R2 den Rest 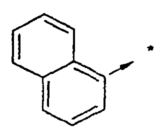 und R5 den Rest 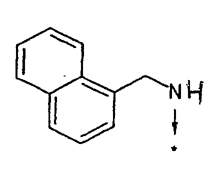 darstellt,
- R2 den Rest 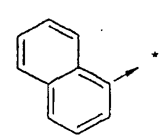 und R5 den Rest 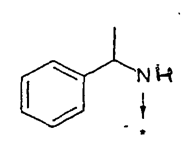 darstellt,
- R2 den Rest 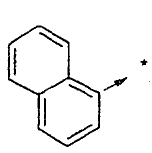 und R5 den Rest 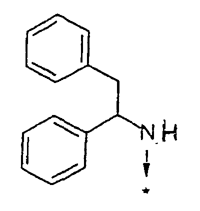 darstellt,
- R2 den Rest 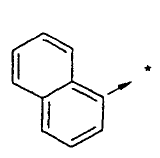 und R5 den Rest 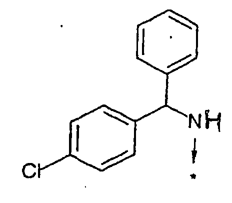 darstellt,
- R2 den Rest 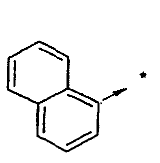 und R5 den Rest 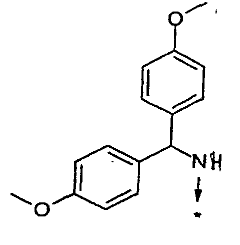 darstellt,
- R2 den Rest 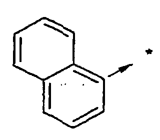 und R5 den Rest 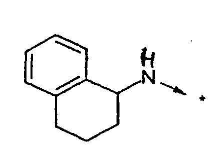 darstellt, oder
- R2 den Rest 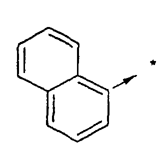 und R5 den Rest 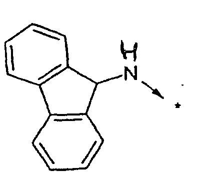 darstellt,
• oder der Formel 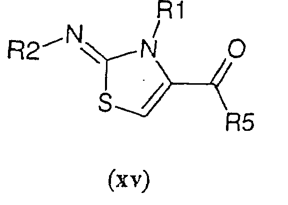 in der
- R1 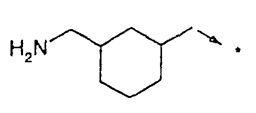 darstellt, R2 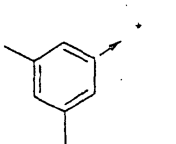 darstellt und R5 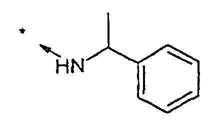 darstellt,
- R1 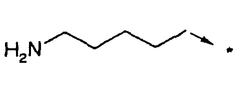 darstellt, R2 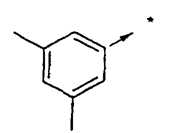 darstellt und R5 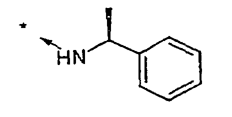 darstellt,
- R1 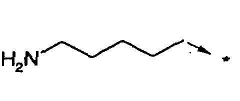 darstellt, R2 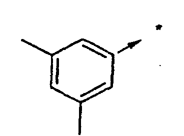 darstellt und R5 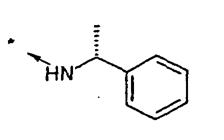 darstellt,
- R1 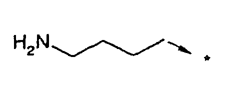 darstellt, R2 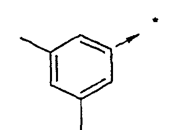 darstellt und R5 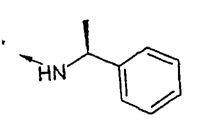 darstellt,
- R1 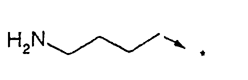 darstellt, R2 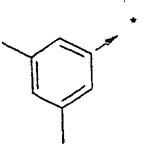 darstellt und R5 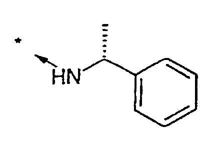 darstellt,
- R1 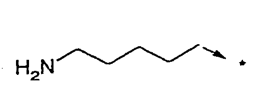 darstellt, R2 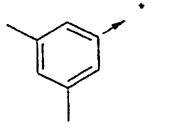 darstellt und R5 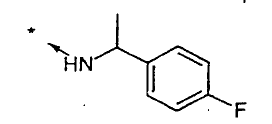 darstellt,
- R1 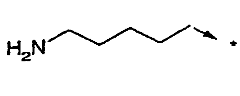 darstellt, R2 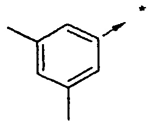 darstellt und R5 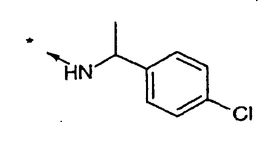 darstellt,
- R1 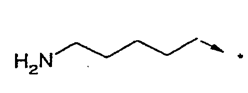 darstellt, R2 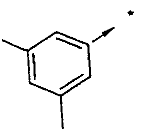 darstellt und R5 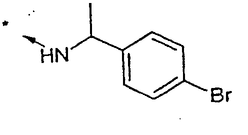 darstellt,
- R1 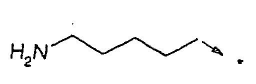 darstellt, R2 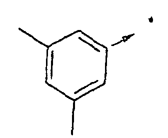 darstellt und R5 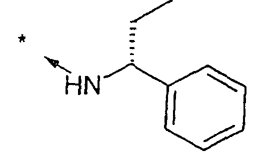 darstellt, oder
- R1 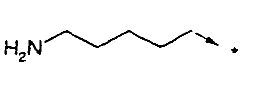 darstellt, R2 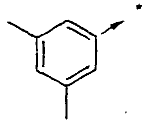 darstellt und R5 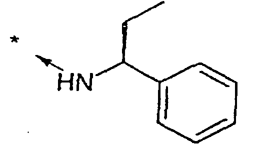 darstellt;
oder ein Salz dieser Verbindungen.

2. Verbindung nach Anspruch 1, **dadurch gekennzeichnet, dass** sie der Formel 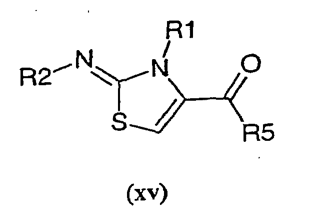 entspricht, in der
- R1 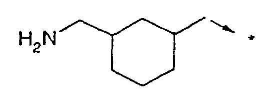 darstellt, R2 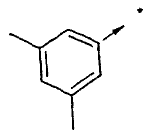 darstellt und R5 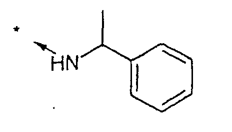 darstellt,
- R1 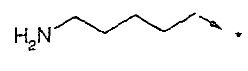 darstellt, R2 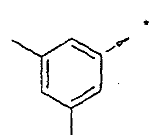 darstellt und R5 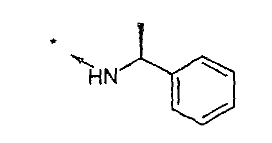 darstellt,
- R1 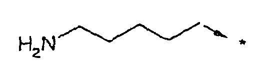 darstellt, R2 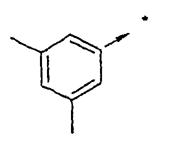 darstellt und R5 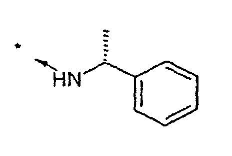 darstellt,
- R1 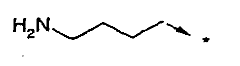 darstellt, R2 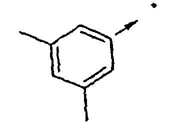 darstellt und R5 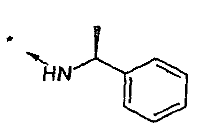 darstellt,
- R1 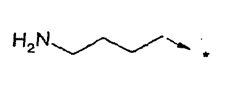 darstellt, R2 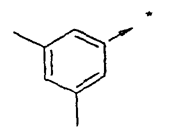 darstellt und R5 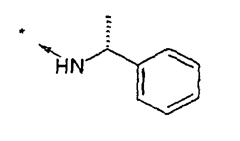 darstellt,
- R1 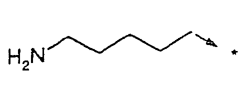 darstellt, R2 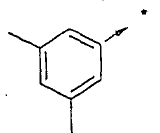 darstellt und R5 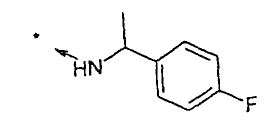 darstellt,
- R1 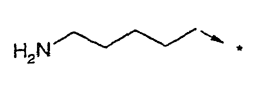 darstellt, R2 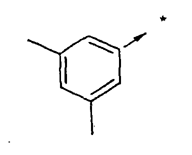 darstellt und R5 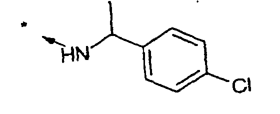 darstellt,
- R1 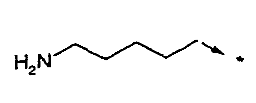 darstellt, R2 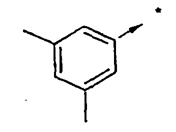 darstellt und R5 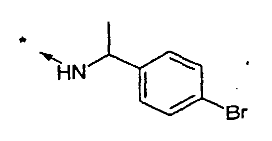 darstellt,
- R1 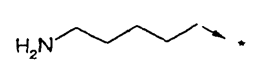 darstellt, R2 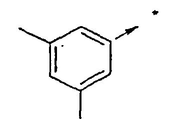 darstellt und R5 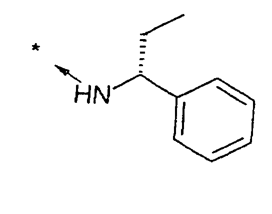 darstellt, oder
- R1 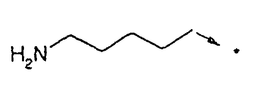 darstellt, R2 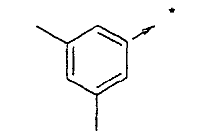 darstellt und R5 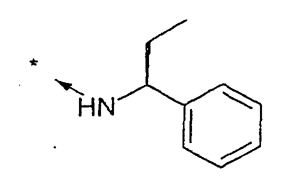 darstellt;
oder ein Salz dieser Verbindungen.

3. Verbindung nach Anspruch 1 oder 2 oder ein pharmazeutisch annehmbares Salz dieser Verbindung in Form eines Medikaments.

4. Pharmazeutische Zusammensetzung, die als Wirkstoff eine Verbindung nach Anspruch 1 oder 2 oder ein pharmazeutisch annehmbares Salz dieser Verbindung umfasst.

5. Verwendung einer Verbindung nach Anspruch 1 oder 2 oder eines pharmazeutisch annehmbaren Salzes dieser Verbindung für die Herstellung eines Medikaments, das für die Behandlung von pathologischen Zuständen oder Krankheiten bestimmt ist, an denen einer (oder mehrere) der Somatostatin-Rezeptoren beteiligt ist (sind).

6. Verwendung nach Anspruch 5, **dadurch gekennzeichnet, dass** die zu behandelnden pathologischen Zustände oder Krankheiten aus der Gruppe ausgewählt sind, die aus den folgenden pathologischen Zuständen oder Krankheiten besteht: Akromegalie, hypophysäre Adenome, Cushing-Krankheit, Gonadotropinome und Prolaktinome, katabolische Nebenwirkungen der Glukokortikoide, insulinabhängiger Diabetes, diabetische Retinopathie, diabetische Nephropathie, X-Syndrom, Dawn-Syndrom, Angiopathie, Angioplastie, Hyperthyroidie, Gigantismus, endokrine gastroenteropankreatische Tumore, darunter das Karzinoidsyndrom, VIPom, Insulinom, Nesidioblastose, Hyperinsulinämie, Glukagonom, Gastrinom und Zollinger-Ellison-Syndrom, GFR-Syndrom sowie akute Blutung der Ösophagusvarizen, Geschwüre, gastroösophagealer Reflux, gastroduodenaler Reflux, Pankreatitis, enterokutane und pankreatische Fisteln, aber auch Diarrhoen, refraktäre Diarrhoen des erworbenen Immundepressionssyndroms, chronische sekretorische Diarrhoe, mit Reizdarmsyndrom verbundene Diarrhoe, mit Chemotherapie induzierte Diarrhoen, mit dem Gastrin-freisetzenden Peptid verbundene Störungen, durch Darmtransplantationen bedingte Krankheiten, portale Hypertonie sowie Hämorrhagien der Varizen bei Zirrhose-Patienten, gastrointestinale Hämorrhagie, Hämorrhagie des Gastroduodenalgeschwürs, Blutung bei implantierten Gefäßen, Crohn-Krankheit, systemische Sklerosen, Dumping-Syndrom, Dünndarmsyndrom, Hypotonie, Sklerodermie und medulläres Thyroidkarzinom, mit einer Zellenhyperproliferation verbundene Krankheiten wie Krebs und insbesondere Brustkrebs, Prostatakrebs, Thyroidkrebs sowie Pankreaskrebs und Kolorektalkrebs, Fibrosen und insbesondere Nierenfibrose, Leberfibrose, Lungenfibrose, Hautfibrose, auch Fibrose des Zentralnervensystems sowie der Nase und mit Chemotherapie induzierte Fibrose und, in anderen therapeutischen Bereichen, Kopfschmerzen einschließlich mit Hypophysentumoren verbundene Kopfschmerzen, Schmerzen, entzündliche Störungen wie Arthritis, Panikattacken, Chemotherapie, Wundvernarbung, Niereninsuffizienz infolge einer Wachstumsverzögerung, Hyperlipidämie, Fettleibigkeit und Wachstumsverzögerung infolge Fettleibigkeit, intrauterinäre Wachstumsverzögerung, Skelettdysplasie, Noonan-Syndrom, Atemstillstand während des Schlafs, Graves-Krankheit, polyzystisches Ovarialsyndrom, pankreatische Pseudozysten und Asziten, Leukämie, Meningiom, krebsbedingte Kachexie, H.pylori-Hemmung, Psoriasis, chronische Abstoßung von Alloimplantaten sowie Alzheimer-Krankheit und schließlich Osteoporose.

7. Verwendung nach Anspruch 6, **dadurch gekennzeichnet, dass** die zu behandelnden pathologischen Zustände oder Krankheiten aus der Gruppe ausgewählt sind, die aus den folgenden pathologischen Zuständen und Krankheiten besteht: Akromegalie, hypophysäre Adenome oder endokrine gastroenteropankreatische Tumore, darunter das Karzinoidsyndrom, und Magendarmblutungen.

8. Verwendung nach Anspruch 6, **dadurch gekennzeichnet, dass** der zu behandelnde pathologische Zustand oder die zu behandelnde Krankheit die Akromegalie ist.

9. Verwendung nach Anspruch 6, **dadurch gekennzeichnet, dass** die zu behandelnden pathologischen Zustände oder Krankheiten aus den hypohysären Adenomen und den endokrinen gastroenteropankreatischen Tumoren ausgewählt sind.

10. Verwendung nach Anspruch 6, **dadurch gekennzeichnet, dass** die zu behandelnden pathologischen Zustände oder Krankheiten aus den Magendarmblutungen ausgewählt sind.

## Claims

1. Compound **characterized in that** it corresponds:
• to the formula 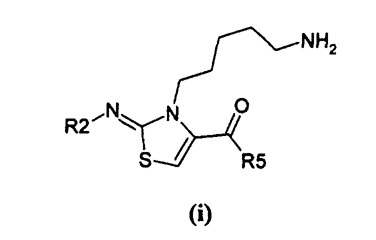 in which:
- R2 represents the 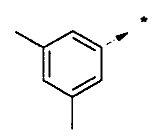 radical and R5 represents the 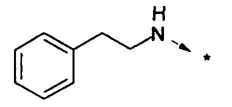 radical,
- R2 represents the 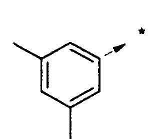 radical and R5 represents the 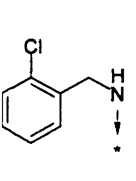 radical,
- R2 represents the 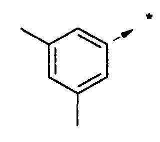 radical and R5 represents the 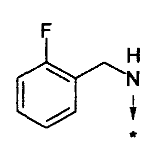 radical,
- R2 represents the 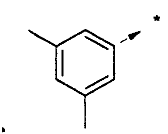 radical and R5 represents the 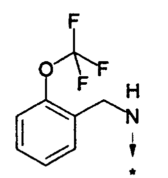 radical,
- R2 represents the 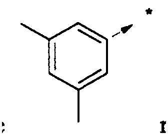 radical and R5 represents the 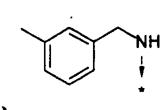 radical, - R2 represents the 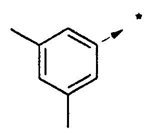 radical and R5 represents the 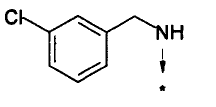 radical,
- R2 represents the 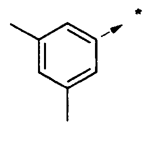 radical and R5 represents the 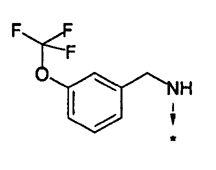 radical,
- R2 represents the 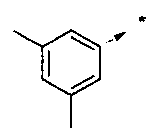 radical and R5 represents the 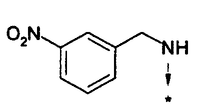 radical,
- R2 represents the 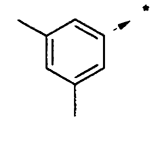 radical and R5 represents the 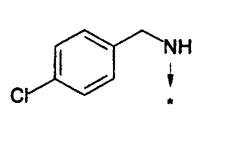 radical,
- R2 represents the 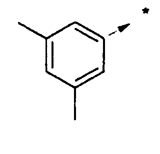 radical and R5 represents the 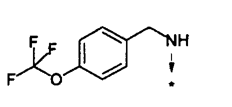 radical,
- R2 represents the 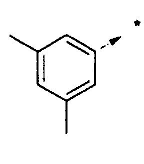 radical and R5 represents the 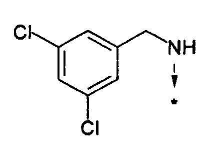 radical,
- R2 represents the 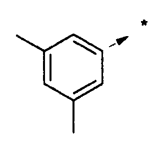 radical and R5 represents the 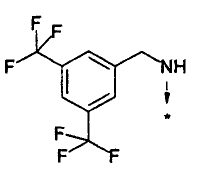 radical,
- R2 represents the 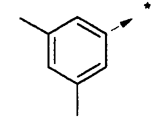 radical and R5 represents the 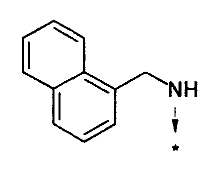 radical,
- R2 represents the 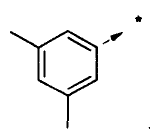 radical and R5 represents the 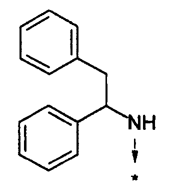 radical,
- R2 represents the 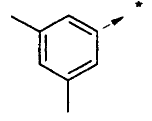 radical and R5 represents the 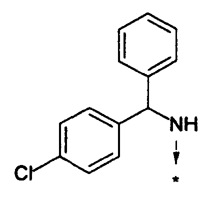 radical,
- R2 represents the 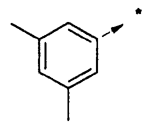 radical and R5 represents the 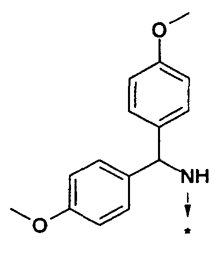 radical,
- R2 represents the 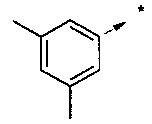 radical and R5 represents the 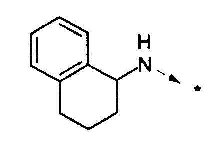 radical,
- R2 represents the 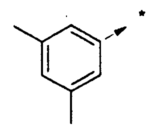 radical and R5 represents the 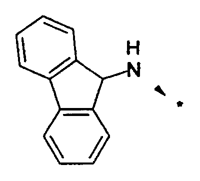 radical,
- R2 represents the 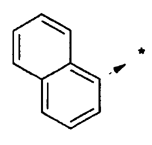 radical and R5 represents the 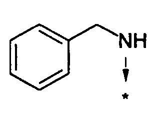 radical,
- R2 represents the 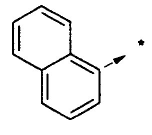 radical and R5 represents the 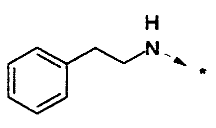 radical,
- R2 represents the 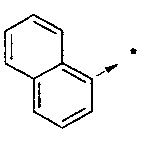 radical and R5 represents the 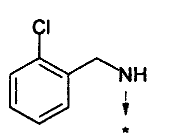 radical,
- R2 represents the 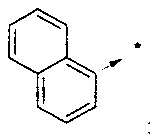 radical and R5 represents the 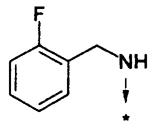 radical,
- R2 represents the 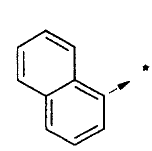 radical and R5 represents the 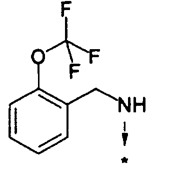 radical,
- R2 represents the 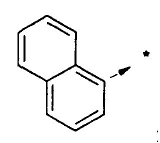 radical and R5 represents the 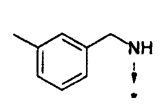 radical,
- R2 represents the 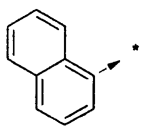 radical and R5 represents the 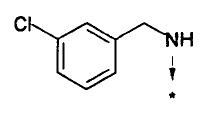 radical,
- R2 represents the 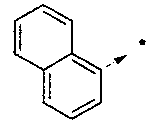 radical and R5 represents the 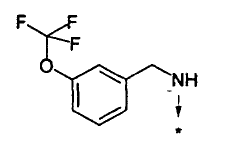 radical,
- R2 represents the 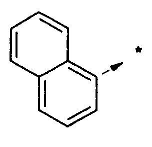 radical and R5 represents the 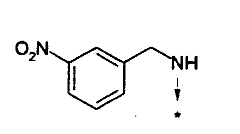 radical,
- R2 represents the 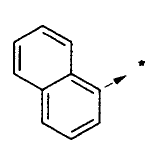 radical and R5 represents the 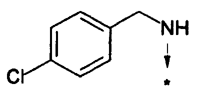 radical,
- R2 represents the 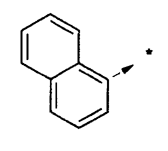 radical and R5 represents the 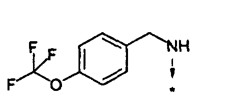 radical,
- R2 represents the 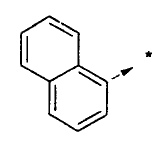 radical and R5 represents the 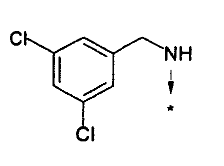 radical,
- R2 represents the 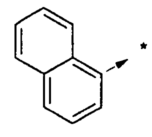 radical and R5 represents the 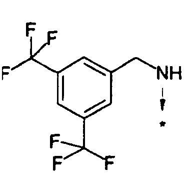 radical,
- R2 represents the 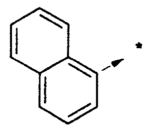 radical and R5 represents the 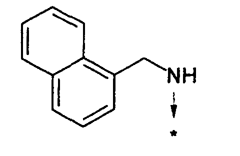 radical,
- R2 represents the 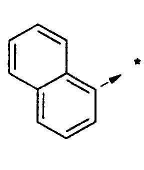 radical and R5 represents the 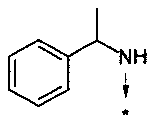 radical,
- R2 represents the 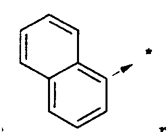 radical and R5 represents the 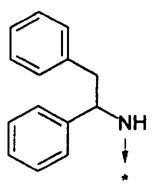 radical,
- R2 represents the 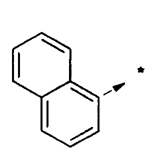 radical and R5 represents the 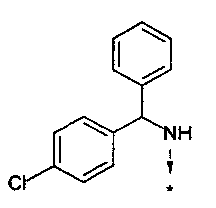 radical,
- R2 represents the 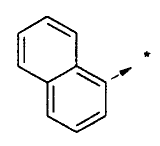 radical and R5 represents the 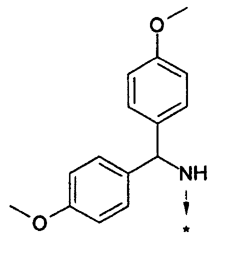 radical,
- R2 represents the 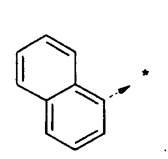 radical and R5 represents the 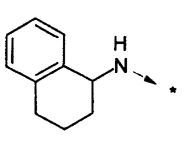 radical, or finally
- R2 represents the 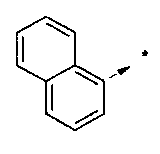 radical and R5 represents the 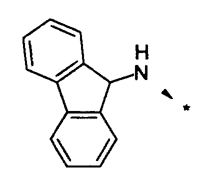 radical;
• or to formula 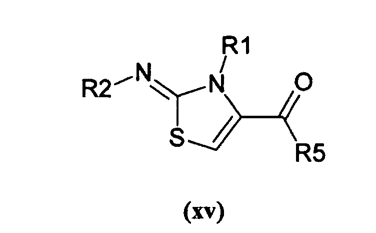 in which:
- R1 represents 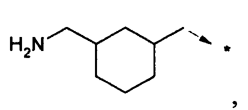 R2 represents 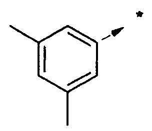 and R5 represents 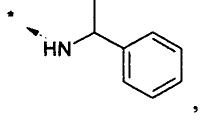
- R1 represents 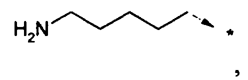 R2 represents 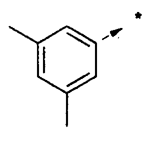 and R5 represents 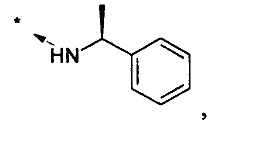
- R1 represents 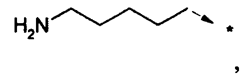 R2 represents 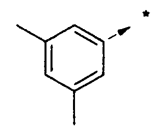 and R5 represents 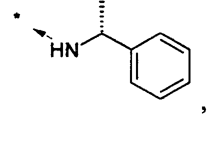
- R1 represents 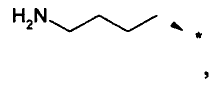 R2 represents 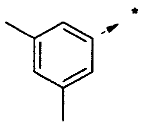 and R5 represents 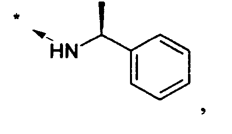
- R1 represents 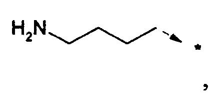 R2 represents 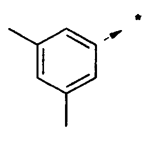 and R5 represents 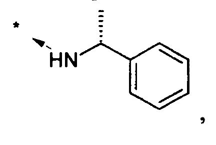
- R1 represents 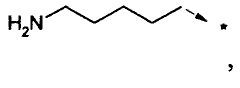 R2 represents 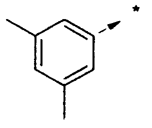 and R5 represents 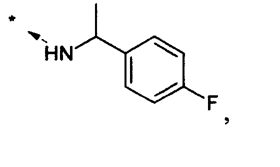
- R1 represents 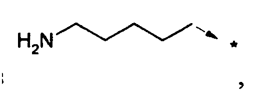 R2 represents 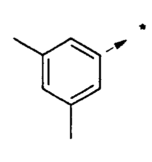 and R5 represents 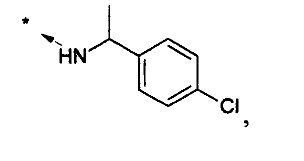
- R1 represents 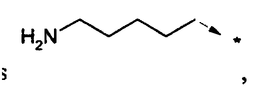 R2 represents 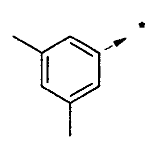 and R5 represents 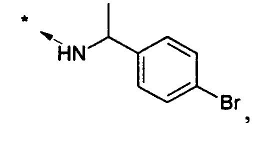
- R1 represents 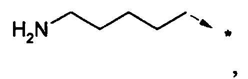 R2 represents 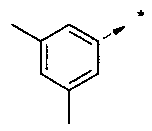 and R5 represents 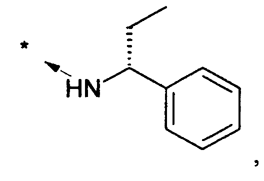 or finally
- R1 represents 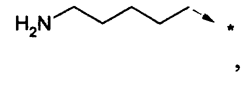 R2 represents 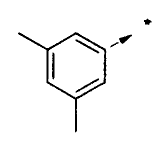 and R5 represents 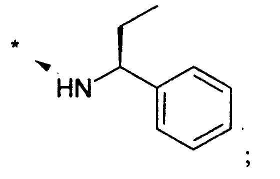
or a salt of one of these compounds.

2. Compound according to claim 1, **characterized in that** it corresponds to the formula 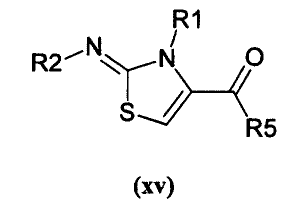 in which:
- R1 represents 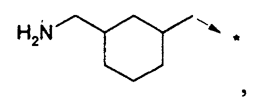 R2 represents 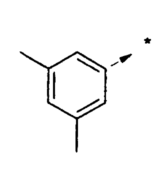 and R5 represents 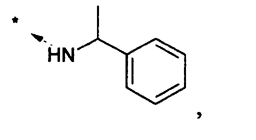
- R1 represents 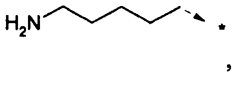 R2 represents 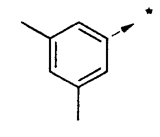 and R5 represents 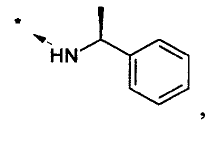
- R1 represents 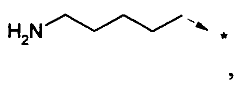 R2 represents 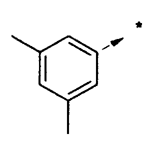 and R5 represents 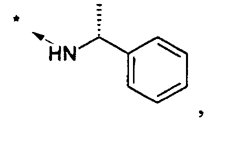
- R 1 represents 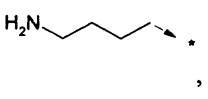 R2 represents 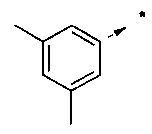 and R5 represents 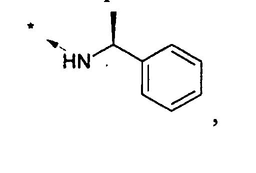
- R1 represents 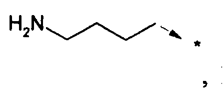 R2 represents 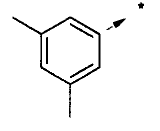 and R5 represents 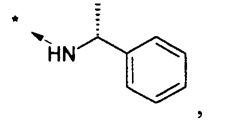
- R1 represents 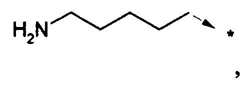 R2 represents 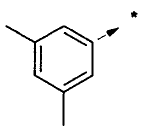 and R5 represents 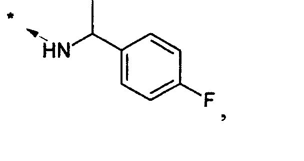
- R1 represents 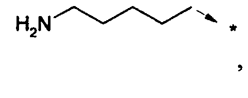 R2 represents 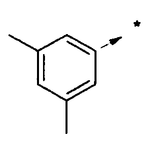 and R5 represents 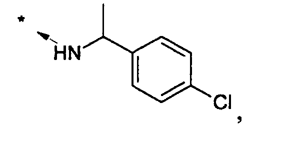
- R1 represents 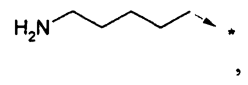 R2 represents 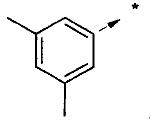 and R5 represents 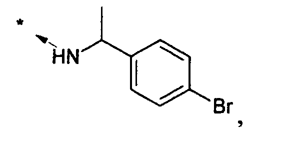
- R1 represents 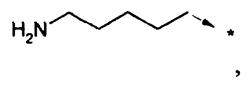 R2 represents 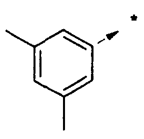 and R5 represents 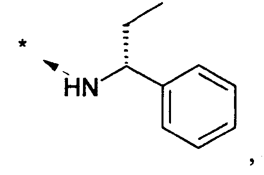 or finally
- R1 represents 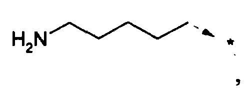 R2 represents 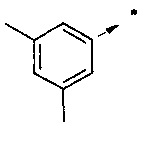 and R5 represents 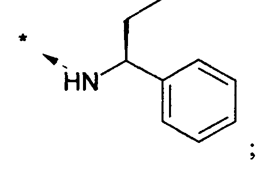
or a salt of one of these compounds.

3. As a medicament, a compound according to claim 1 or 2 or a pharmaceutically acceptable salt of said compound.

4. Pharmaceutical composition comprising, as active ingredient, a compound according to claim 1 or 2 or a pharmaceutically acceptable salt of said compound.

5. Use of a compound according to claim 1 or 2 or of a pharmaceutically acceptable salt of said compound for preparing a medicament intended to treat the pathological states or diseases in which one (or more) of the somatostatin receptors is (are) involved.

6. Use according to claim 5, **characterized in that** the pathological states or the diseases to be treated are chosen from the group comprising the following pathological states or diseases: acromegalia, hypophyseal adenomas, Cushing's disease, gonadotrophinomas and prolactinomas, catabolic side-effects of glucocorticoids, insulin dependent diabetes, diabetic retinopathy, diabetic nephropathy, syndrome X, dawn phenomenon, angiopathy, angioplasty, hyperthyroidism, gigantism, endocrinic gastroenteropancreatic tumors including carcinoid syndrome, VIPoma, insulinoma, nesidioblastoma, hyperinsulinemia, glucagonoma, gastrinoma and Zollinger-Ellison's syndrome, GRFoma as well as acute bleeding of the esophageal varices, ulcers, gastroesophageal reflux, gastroduodenal reflux, pancreatitis, enterocutaneous and pancreatic fistulae but also diarrheas, refractory diarrheas of acquired immunodeficiency syndrome, chronic secretary diarrhea, diarrhea associated with irritable bowel syndrome, diarrheas induced by chemotherapy, disorders linked with gastrin releasing peptide, secondary pathologies with intestinal grafts, portal hypertension as well as hemorrhages of the varices in patients with cirrhosis, gastro-intestinal hemorrhage, hemorrhage of the gastroduodenal ulcer, bleeding of grafted vessels, Crohn's disease, systemic scleroses, dumping syndrome, small intestine syndrome, hypotension, scleroderma and medullar thyroid carcinoma, illnesses linked with cell hyperproliferation such as cancers and more particularly breast cancer, prostate cancer, thyroid cancer as well as pancreatic cancer and colorectal cancer, fibroses and more particularly fibrosis of the kidney, fibrosis of the liver, fibrosis of the lung, fibrosis of the skin, also fibrosis of the central nervous system as well as that of the nose and fibrosis induced by chemotherapy, and in other therapeutic fields, cephaleas including cephalea associated with hypophyseal tumors, pain, inflammatory disorders such as arthritis, panic attacks, chemotherapy, cicatrization of wounds, renal insufficiency resulting from delayed development, hyperlipidemia, obesity and delayed development linked with obesity, delayed uterine development, dysplasia of the skeleton, Noonan's syndrome, sleep apnea syndrome, Graves' disease, polycystic disease of the ovaries, pancreatic pseudocysts and ascites, leukemia, meningioma, cancerous cachexia, inhibition of H pylori, psoriasis, chronic rejection of allografts as well as Alzheimer's disease and finally osteoporosis.

7. Use according to claim 6, **characterized in that** the pathological states or diseases to be treated are chosen from the group comprising the following pathological states or diseases: acromegalia, hypophyseal adenomas or endocrinic gastroenteropancreatic tumors including carcinoid syndrome, and gastrointestinal bleeding.

8. Use according to claim 6, **characterized in that** the pathological state or disease to be treated is acromegalia.

9. Use according to claim 6, **characterized in that** the pathological states or diseases to be treated are chosen from hypophyseal adenomas and endocrinic gastroenteropancreatic tumors.

10. Use according to claim 6, **characterized in that** the pathological states or diseases to be treated are chosen from gastrointestinal bleeding.
